# EUROPEAN PATENT SPECIFICATION

(11) **EP 1 218 514 B1**
(45) Date of publication and mention of the grant of the patent: **26.04.2006**
(21) Application number: 00964541.7
(22) Date of filing: 06.10.2000
(51) Int. Cl.: C12N 15/36, C07K 14/02, A61K 39/12, A61P 37/02, C12N 15/62

(54) **DESIGNING IMMUNOGENS**
DESIGN VON IMMUNOGENE
CONCEPTION D'IMMUNOGENES

(30) Priority: 08.10.1999 GB 9923902; 30.03.2000 GB 0007789
(43) Date of publication of application: 03.07.2002
(73) Proprietor: Celltech Pharma Europe Limited, Slough, Berkshire SL1 3WE (GB)
(72) Inventor: JONES, C. Elan Corp., Dublin 2 (IE); BACON, A. Lipoxen Technologies Limited, London WC1N 1AX (GB); DOUCE, G. Divisin of Infection & Immunity, IBLS, Glasgow, G12 8QQ (GB); PAGE, M. Apovia AG, 82152 Martinsried München (DE)
(74) Representative: Campbell, Patrick John Henry
(86) International application number: PCT/GB2000/003857
(87) International publication number: WO 2001/027281

(56) References cited:
- EP-A- 0 421 635
- WO-A-94/01132
- HERNANDEZ H.J. & STADECKER M.J.: "Elucidation and role of critical residues of immunodominant peptide associated with T cell-mediated parasitic disease" J. IMMUNOL., vol. 163, 1 October 1999 (1999-10-01), pages 3877-3882, XP002157914
- CONSTANT S L ET AL: "INDUCTION OF TH1 AND TH2 CD4+ T CELL RESPONSES: THE ALTERNATIVE APPROACHES" ANNUAL REVIEW OF IMMUNOLOGY, 1997, XP002060443
- MILICH D.R. ET AL.: "The Hepatitis B virus Core and e antigens elicit different Th subsets: antigen structure can affect Th cell phenotype." J. VIROLOGY, vol. 71, no. 3, March 1997 (1997-03), pages 2192-2201, XP002157915
- SCHOEDEL F ET AL: "THE POSITION OF HETEROLOGOUS EPITOPES INSERTED IN HEPATITIS B VIRUS CORE PARTICLES DETERMINES THEIR IMMUNOGENICITY" JOURNAL OF VIROLOGY,US,NEW YORK, US, vol. 66, no. 1, January 1992 (1992-01), pages 106-114, XP000891477 ISSN: 0022-538X
- DALUM I. ET AL: 'Induction of cross-reactive antibodies against a self protein by immunization with a modified self protein containing a foreign T cell epitope' MOLECULAR IMMUNOLOGY vol. 34, no. 16-17, 1997, pages 1113 - 1120, XP000910038

## Description

The invention relates to the design of protein immunogens, for example for use in immunotherapy of diseases.

### Background to the invention

Much research is being done to try to find treatments for diseases by targeting the immune system at the causes of the diseases. Such diseases include cancer and chronic viral hepatitis. In the case of cancer, the aim is to induce the patient's immune system to reject the tumour. In the case of chronic viral hepatitis, the aim is to induce the immune system to clear infected virus from liver cells.

Chronic viral hepatitis can be caused by hepatitis B virus (HBV) or hepatitis C virus (HCV). In about 90-95% of cases of infection by HBV, the virus is cleared by the infected patient in a relatively short time. However, in the remaining 5-10% of cases the disease becomes chronic, i.e. it persists in the patient for a long period of time. Patients are said to be chronic carriers of HBV if the viral DNA persists for longer than 10 weeks, the hepatitis B e antigen (HBeAg) persists for more than 12 weeks or the hepatitis B surface antigen (HBsAg) persists for more than 6 months.

It is believed that patients with chronic hepatitis have an unbalanced T-helper (Th) cell response, and that this may at least partly explain their failure to clear the virus. There are two types of T-helper cells, Th1 and Th2 cells. Th1 cells promote cytotoxic T cell (CTL) activation and Th2 cells promote antibody production by B cells. Cytotoxic T cells are able to help clear the viral infection; in the case of hepatitis, it is believed that some cytotoxic T cells clear virus from liver cells directly and others clear virus by means of antiviral cytokines such as interferon gamma (IFNγ) and tumour necrosis factor alpha (TNFα). Antibodies by themselves are not able to clear intracellular hepatic viral infection because they are not able to enter the cell and mediate destruction of the virus. Thus, activation of Th1 cells and consequent activation of cytotoxic T cells is believed to be essential for the successful clearance of chronic hepatitis. In patients with chronic viral hepatitis, Th2 cell responses to HBV antigens dominate and the number of activated Th1 cells is believed to be inadequate to clear the virus. A goal in the treatment of the disease is therefore to switch patients' Th responses from a Th2 dominated response to a Th1 dominated response.

In the case of cancer, it is believed that enhancement of the Th1 response to tumour antigens may help the immune system to destroy the tumour by means of CTL activation. It is also believed that allergic diseases in which Th2 responses dominate, such as excema and atopic allergies, may be treated by enhancing the Th1 response.

### Summary of the invention

The invention provides a method for designing a protein immunogen, which comprises
(a) modifying the amino acid sequence of the immunogen, and
(b) determining whether the Th cell response to the modified immunogen is of the Th1 type or the Th2 type.

The method is particularly useful in the design of a protein immunogen for use in immunotherapy of a disease because it allows the immunogen to be designed such that it induces the type of Th response which is required to treat the disease.

### Detailed description of the invention

### The protein

The protein modified according to the invention may be any protein which induces a Th cell response in a mammal. Typically, the protein is one which is useful as a carrier for presenting heterologous epitopes to the immune system. Epitopes by themselves often have low immunogenicity, but the level of immunogenicity can be increased by presenting epitopes on carrier proteins. Classically, epitopes have been chemically linked to proteins such as keyhole limpet hemocyanin, bovine serum albumin and mycobacterial heat shock proteins. More recently, fusion proteins comprising epitopes presented on carrier proteins have been made using recombinant DNA technology.

A preferred category of proteins to be modified according to the invention are particle-forming proteins, particularly proteins which form the capsids and envelopes of viruses. Such proteins are often highly immunogenic and can be engineered to present heterologous epitopes to the immune system. This choice of protein can take advantage of the intrinsic ability of viral capsid and envelope proteins to self-assemble into highly organised particles, frequently without need for further viral components. The particles lack the complete viral genome and are non-pathogenic. They comprise multiple copies of the protein, for example from 20 to 500 or 50 to 300 copies. They can often be expressed in large quantities by recombinant DNA techniques, and are often easily purified owing to their particulate structure. Examples include the core and surface antigens of HBV (HBcAg and HBsAg), retroviral Gag proteins, phage coat proteins, the haemagglutinin antigen (HA) of influenza virus, and proteins of hepatitis A virus (HAV), of HCV and of poliovirus.

A particularly preferred protein is HBcAg, which forms the capsid of HBV. This protein has long been known to be highly immunogenic, and induces strong antibody and cellular immune responses. It has been shown to protect against HBV challenge in the chimpanzee model of the disease.

The HBcAg protein is 21 kDa and consists of 183 to 185 amino acids (aa) depending on the HBV subtype. It is the product of the HBV core (or C) open reading frame. A second, related protein is HBeAg, which does not normally assemble into particles but which consists of the same sequence as HBcAg with a truncation of the C-terminus at position 149 and an extension at the N-terminus. HBcAg has been expressed in a variety of heterologous systems such as bacteria, yeast, insect cells and mammalian cells. It self-assembles into particles in the absence of other viral components. The particles are 27 nm in diameter and are spiked, with one form having 120 spikes and 240 polypeptide chains per particle and another form having 90 spikes and 180 polypeptide chains per particle.

The inventors' work has revealed information about the effect of modifications in the HBcAg sequence on the subclass of Th response induced by the HBcAg. It has surprisingly been found that even small modifications in the sequence of HBcAg can switch the type of Th cell response against the protein and that the precise nature of the modification can have a dramatic effect on the type of Th cell response induced by the modified protein. For example, the inventors' work indicates that:
- Modification of the sequence in the e1 loop of HBcAg can have a dramatic effect on the Th subclass response against other parts of HBcAg. Unmodified HBcAg induces a Th1 dominated IgG subclass response against non-e1 loop epitopes. Insertion of a pre-S1 sequence of HBV (amino acids 20-47) into the e1 loop causes the Th1 response against the non-e1 loop HBcAg epitopes to switch towards a Th2 (IgG1) response, whereas inserting a pre-S2 sequence (amino acids 140-174) into the same loop causes no such switch.
- Truncation of the HBcAg sequence at the C-terminus can change the Th subclass response against the e1 loop or a heterologous epitope inserted in the e1 loop from a Th1 response to a Th2 response. For example, it has been found that a preparation of full length HBcAg or full length HBcAg with a pre-S1 sequence (amino acids 20-47) inserted in the e1 loop ("Core-S1") induces a Th1 dominated response against the e1 loop or pre-S1 insert in the e1 loop. However, a preparation of HBcAg truncated at position 146 or Core-S1 truncated at position 154 induces a Th2 dominated response against the e1 loop or pre-S1 insert in the e1 loop. This indicates that it is important to retain the C-terminus of HBcAg in designing immunogens for treatment of diseases in which it is desirable to induce a Th1 response, such as chronic viral hepatitis.

The reason why the presence of the C-terminus of HBcAg tends to favour a Th1 response is not known, but the inventors have some theories (to which they do not wish to be bound).

One theory is that the presence of the cysteine residue which is at the C-terminus of HBcAg is important for inducing a Th1 response. In the experiments described in the Examples, those constructs which contained the C-terminal cysteine induced a Th1 response against the e1 loop or inserted epitope and those which did not contain the cysteine induced a Th2 response against the loop or epitope. The cysteine participates in the formation of disulfide bonds and may therefore help to stabilise the particle. Particles containing full length protein with the C-terminal cysteine may be more resistant to endosomal processing of the protein than particles containing truncated protein. This may alter the Th epitopes that are produced following particle processing.

Another theory is that the presence of DNA bound to the C-terminus of HBcAg (positions downstream of aa 146) may affect the subclass response against the e1 loop region. The inventors found that, for example, preparations of full length Core-S1 contain higher amounts of DNA than Core-S1 truncated at position 154 and induce Th1 and Th2 IgG subclass responses against the pre-S1 insert, respectively. Thus, the presence of DNA may favour a Th1 dominated response.

### The modification

The protein sequence may be modified by a substitution, insertion, deletion or extension. The size of insertion, deletion or extension may, for example, be from 1 to 200 aa, from 3 to 100 aa or from 6 to 50 aa. Similarly, the number of amino acids replaced in a substitution may, for example, be from 1 to 200, from 3 to 100, from 6 to 50, from 1 to 20 or from 1 to 10.

An extension may be at the N- or C-terminus of the protein. A deletion may be at the N-terminus, C-terminus or at an internal site of the protein. Substitutions may be made at any position in the protein sequence. Insertions may also be made at any point in the protein sequence, but are typically made in surface-exposed regions of the protein.

More than one modification may be made to a given protein. Thus, it is, for example, possible to make a terminal extension or deletion to a protein and also an internal insertion. In the case of HBcAg, a deletion may be made in the C-terminal region and an insertion may be made in the e1 loop.

The modifications are generally chosen so as not to destroy the conformation of the protein, and in the case of particle-forming proteins the modifications are generally chosen so as not to destroy the particle-forming ability of the protein. Such modifications are made at sites in the protein which are not important for maintainance of its conformation, for example at the termini or in internal surface-exposed regions. Many proteins have internal regions which are known to be surface exposed and can tolerate modifications without destruction of the conformation of the protein. For example, the e1 loop of HBcAg can tolerate insertions of e.g. from 1 to 100 amino acids without destroying the particle-forming ability of the protein.

The e1 loop of HBcAg is at positions 68 to 90, and a heterologous epitope may be inserted in this region. Preferably, the epitope is inserted in the region from positions 69 to 90, 71 to 90 or 75 to 85. Most preferred is to insert the epitope between amino acid residues 79 and 80 or between residues 80 and 81. When a heterologous epitope is inserted, the entire sequence of the carrier protein may be maintained, or alternatively part of the carrier protein sequence may be deleted and replaced by the heterologous sequence. Thus, in the case of HBcAg, amino acid residues 69 to 90, 71 to 90 or 75 to 85 may be replaced by a heterologous epitope. Where a heterologous epitope replaces sequences of the carrier protein, the epitope is generally not shorter than the sequence that it replaces.

A C-terminal truncation of HBcAg will generally not go beyond aa 144 because if any further truncation is made particles may not form. Thus, the deleted amino acids may, for example, comprise aa 144 to the C-terminal aa (aa 183 or 185), aa 146 to the C-terminal aa, aa 154 to the C-terminal aa, aa 164 to the C-terminal aa or aa 172 to the C-terminal aa. The C-terminus of HBcAg binds DNA, and truncation of the C-terminus therefore reduces or completely removes DNA from preparations of HBcAg and HBcAg hybrid proteins.

In some circumstances, for example where a Th response is desired, it may be preferable to retain the C-terminus of HBcAg. Thus, an HBcAg immunogen designed according to the invention may preferably contain the C-terminal sequence, in particular the C-terminal cysteine.

The C-terminal cysteine is typically preceded by the sequence immediately upstream of the residue in HBcAg. The preceding HBcAg sequence may comprise from 1 to 7 residues, i.e. 1, 2, 3, 4, 5, 6 or 7 residues. Thus, the C-terminus of the protein of the invention may comprise the sequence Gin Cys, Ser GIn Cys, Glu Ser Gln Cys, Arg Glu Ser Gln Cys, Ser Arg Glu Ser Gln Cys, Gln Ser Arg Glu Ser Gln Cys or Ser Gln Ser Arg Glu Ser Gln Cys. However, the Cys residue may not be the one from HBcAg; in this case, an immunogen may be constructed by truncating the HBcAg sequence and replacing the truncated sequence with another sequence including a Cys residue and optionally an epitope from a protein other than HBcAg. The Cys residue is typically located at the extreme C-terminal end of the immunogen but it may be a number of amino acid residues from the extreme C-terminal end. For example, it may be from 1 to 20, from 1 to 10 or from 1 to 5 residues from the C-terminus. In any event, the Cys residue must be able to form a disulfide bond.

The modification may be made by any technique available in the art, including chemical modification and modification using recombinant DNA technology. The use of recombinant DNA technology is generally preferred because it allows the site of the modification to be precisely defined and large quantities of the modified protein can be produced in a heterologous host.

### Heterologous epitopes

As indicated above, a preferred modification to the protein is insertion of a heterologous epitope. This allows an immune response to be produced not only against the carrier protein but also against the heterologous epitope.

An important finding of the invention is that the choice of epitope affects the Th response induced by the carrier protein. As mentioned above, it has for example been found that inserting amino acids 20-47 of the pre-S1 region of HBV into the e1 loop region of HBcAg causes the Th response (as indicated by IgG subclass) against the HBcAg antigen to switch from a Th1 type response to a Th2 type response, whereas inserting amino acids 140-174 of the pre-S2 region of HBV into the same loop causes no such switch.

The choice of epitope depends on the type of Th cell response that it is wished to induce and the disease that it is wished to treat. The epitope may be from a protein that is associated with a disease that can be treated by inducing a Th cell response. Such diseases include diseases caused by infection by pathogenic organisms, cancers and allergies. The pathogenic organism may, for example, be a virus, a bacterium or a protozoan.

In the case of treatment of diseases caused by infection by a pathogenic organism, the epitope is typically from an antigen of the organism. In the case of treatment of cancer, the epitope is typically from an antigen expressed by tumour cells, such as antigens encoded by oncogenes. In the case of treatment of an allergy, the epitope is typically from a protein that induces an allergic reaction, for example house dust mite allergen.

A "heterologous" epitope is an epitope that is not normally located at the position at which it is located in the modified protein; it is generally from a protein different from the protein modified to carry the epitope but may be from a different location in the same protein. The epitope comprises a sequence of amino acids which raises an immune response. The epitope may be conformational or linear. It may be, for example, in a sequence of from 6 to 100 aa, from 6 to 50 aa or from 6 to 20 aa. It may be a T cell or a B cell epitope. If it is a T cell epitope, it may induce a Th1 or a Th2 response. It may induce a switch in the type of Th cell response against the carrier protein or it may not induce any such switch. More than one copy of an epitope may be added to the carrier protein; for example, from 2 to 8 epitopes may be added.

Examples of pathogens whose epitopes may be inserted include hepatitis A virus (HAV), HBV, HCV, influenza virus, foot-and-mouth disease virus, poliovirus, herpes simplex virus, rabies virus, feline leukemia virus, human immunodeficiency virus type 1 (HIV1), human immunodeficiency virus type 2 (HIV2), simian immunodeficiency virus (SIV), human rhinovirus, dengue virus, yellow fever virus, human papilloma virus, respiratory syncytial virus, *Plasmodium falciparum* (a cause of malaria), and bacteria such as *Mycobacteria, Bordetella, Salmonella, Escherichia, Vibrio, Haemophilus, Neisseria, Yersinia* and *Brucella.* Specifically, the bacterium may be *Mycobacterium tuberculosis -* the cause of tuberculosis; *Bordetella pertussis* or *Bordetella parapertussis -* causes of whooping cough; *Salmonella typhimurin -* the cause of salmonellosis in several animal species; *Salmonella typhi* - the cause of human typhoid; *Salmonella enteritidis -* a cause of food poisoning in humans; *Salmonella choleraesuis -* a cause of salmonellosis in pigs; *Salmonella dublin -* a cause of both a systemic and diarrhoeal disease in cattle, especially of new-born calves; *Escherichia coli -* a cause of food poisoning in humans; *Haemophilus influenzae -* a cause of meningitis; *Neisseria gonorrhoeae -* a cause of gonnorrhoeae; *Yersinia enterocolitica -* the cause of a spectrum of diseases in humans ranging from gastroenteritis to fatal septicemic disease; or *Brucella abortus -* a cause of abortion and infertility in cattle and a condition known as undulant fever in humans.

Examples of candidate epitopes for use in the invention include epitopes from the following antigens: the HIV antigens gp 120, gp 160, gag, pol, Nef, Tat and Rev; the malaria antigens CS protein and Sporozoite surface protein 2; the influenza antigens HA, NP and NA; the herpes virus antigens EBV gp340, EBV gp85, HSV gB, HSV gD, HSV gH, HSV early protein product, cytomegalovirus gB, cytomegalovirus gH, and IE protein gP72; the human papilloma virus antigens E4, E6 and E7; the respiratory syncytial virus antigens F protein, G protein, and N protein; the pertactin antigen of *B.pertussis* and the tumor antigens carcinoma CEA, carcinoma associated mucin, carcinoma P53, melanoma MPG, melanoma P97, MAGE antigen, carcinoma Neu oncogene product, prostate specific antigen (PSA), prostate associated antigen, ras protein, and myc.

In the case of HBV, the epitope may be from the pre-S1 region, the pre-S2 region, the S region or core antigen. It is possible to insert the whole of the pre-S1 and/or the whole of the pre-S2 region into the carrier protein, but generally only a part of one of the regions is inserted. The inserted part is typically at least 6 amino acids in length, for example from 6 to 120 aa, 8 to 80 aa or 10 to 40 aa. The insert may include, for example, the residues at pre-S1 positions 1-9, 10-19, 20-29, 30-39, 40-49, 50-59, 60-69, 70-79, 80-89, 90-99, 100-109 or 110-119 or the residues at pre-S2 positions 120-129, 130-139, 140-149, 150-159, 160-169 or 170-174. Particularly preferred fragments are those corresponding to pre-S1 residues 20-47 and pre-S2 residues 140-174.

### Determining the effect of a modification

The effect of a modification on the Th response induced by the protein may be determined using assays known in the art. Th1 and Th2 responses are each known to be associated with production of particular subclasses of IgG and with production of particular cytokines.

In mice, a Th1 response is associated with production of IgG2a, interferon gamma (IFNγ) and interleukin 2 (IL2), and a Th2 response is associated with production of IgG1, IL4, IL5, IL6 and IL10. Thus, in mice the induction of a Th1 response may be detected by detecting IgG2a, IFNγ and/or IL2, and induction of a Th2 response may be detected by detecting IgG1, IL4, IL5, IL6 and/or IL10.

In humans, a Th1 response is associated with production of IgG1, IgG3, IFNγ and IL-2, and a Th2 response is associated with the production of IgG2, IgG4, IL-4, IL-5, IL-6, IL-13 and possibly IL-10. Thus, in humans the induction of a Th1 response may be detected by detecting IgG1 and/or IgG3, and the induction of a Th2 response may be detected by detecting IgG2 and/or IgG4.

A preferred way of determining the class of Th response is to carry out ELISA assays to detect the Ig subclass response, for example to detect whether a mouse response is IgG1 or IgG2a dominated. The IgG subclass can be determined in such assays by the use of antibodies specific for particular subclasses of IgG, for example anti-IgG1 and anti-IgG2a antibodies (which are commercially available), conjugated to detectable labels.

The modification to the protein may induce a switch from Th1 to Th2 production or vice versa. Alternatively, the modification may not induce any switch. Thus, the method of the invention may be used to identify modifications which produce switching or to identify modifications which do not cause switching. The Th response to the modified protein may be compared with that of the unmodified protein. For example, in the case of use of modified HBcAg in the treatment of chronic hepatitis, it is desirable to maintain the Th1 response induced by the unmodified (full length) protein. Thus, the method of the invention can be used in the design of immunogens useful in therapy of chronic hepatitis by identifying modifications which do not cause Th type switching. A Th1 response may be indicative that an immunogen is useful in the treatment of diseases such as chronic hepatitis (e.g. chronic HBV or chronic HCV), cancer or allergies.

The subclass dominance may be calculated by measuring the ratio of a marker of a Th2 response to a marker of a Th1 response (the Th2:Th1 ratio). A Th2:Th1 ratio of >2 indicates Th2 dominance and a ratio of <0.5 indicates Th1 dominance. In the method of the invention, a ratio of >2, >3, >5 or >10 may be taken as an indication of Th2 dominance, whereas a ratio of <0.5, <0.3, <0.2 or <0.1 may be taken as an indication of Th1 dominance.

The method of the invention may comprise the additional step of determining whether the modified protein immunogen forms particles comprising multiple copies of the protein immunogen. It is generally preferred that the immunogen does form particles because epitopes tend to induce stronger immune responses when presented on particles, but the immunogen may be in non-particulate form. Size exclusion chromatography (SEC) may be used to gain an indication of whether particles are formed.

The method of the invention may also comprise determining whether the immunogen has bound nucleic acid (e.g. DNA). It is generally desirable to avoid the presence of nucleic acid because it is generally regarded as an impurity, to be avoided in pharmaceutical compositions for administration to humans or animals. The presence or absence of DNA may be detected using agarose gel electrophoresis and/or spectrophotemetry.

Thus, the invention may be used to design an immunogen which not only induces a particular Th subclass response, but also an immunogen which has other desirable properties such as a particulate structure and/or an absence of detectable nucleic acid. In the design of an immunogen for therapy of disease such as chronic hepatitis, a preferred immunogen induces a Th1 response, has a particulate structure and/or is free of detectable nucleic acid. In the case of HBV core antigen, retention of the C-terminal cysteine is preferred.

### Pharmaceutical compositions

An immunogen designed according to the invention may be formulated into a pharmaceutical composition with a pharmaceutically acceptable inert carrier or diluent.

Ways of formulating immunogens for administration to humans and animals are known to persons skilled in the art. The quantity of immunogen administered may ultimately be at the discretion of the physician and may depend on factors such as the disease to be treated and the patient to be treated. However, the dose will typically be in a range of from 1 µg to 250 µg per dose, for example from 10 µg to 100 µg.

The pharmaceutical composition may be administered in one dose only, but generally will be administered in multiple doses. For example, from 2 to 32 or from 4 to 16 doses may be given. The time period between doses may, for example, be from 1 week to 4 months.

More than one immunogen designed according to the invention may be administered to a patient. Furthermore, an immunogen designed according to the invention may be used in combination with one or more other compositions. For example, in the treatment of chronic HBV an immunogen may be used in combination with interferon alpha, Lamivudine™, or another immunotherapeutic agent such as Hepacare™ (formerly known as Hepagene™). The immunogen designed according to the invention and the other composition may be administered simultaneously or sequentially.

The pharmaceutical composition of the invention will generally be prepared as an injectable, either as a solution or a suspension, e.g. in saline. Solid forms suitable for solution or suspension in liquid prior to injection may also be prepared. The pharmaceutical composition will generally include an adjuvant, for example aluminum hydroxide or aluminum phosphate.

The following Examples serve to illustrate the invention.

### Brief Description of the Drawings

**Figure 1a** is a restriction endonuclease map of plasmid pGA/S1-7 and **Figure 1b** is a restriction endonuclease map of plasmid ptrc/core/S1.
**Figure 2** shows an SDS-PAGE analysis of Core-S1 and Core-S1₁₅₄ (immunogens described in the Examples). Pre-cast gels (NuPAGE™ MES; Novex) were used. Protein bands were stained with Coomassie Colloidal Blue (Sigma). Lanes 1 and 3 contain sea-blue protein ladder (5 µl) (Novex), lane 2 contains Core-S1(6.1 µg) and lane 4 contains Core-S1₁₅₄ (0.7 µg).
**Figure 3** is a restriction endonuclease map of plasmid pTCST₁₅₄.
**Figure 4** shows an SDS-PAGE analysis of Core and Core₁₄₆ (immunogens described in the Examples). Pre-cast gels (NuPAGE™ MES; Novex) were used. Protein bands were stained with Coomassie Colloidal Blue (Sigma). Lanes 1 and 3 contain sea blue protein ladder (5 µl) (Novex), lane 2 contains Core (1.4 µg) and lane 4 contains Core₁₄₆ (7.9 µg).
**Figure 5** shows the results of size exclusion chromatography (SEC) analysis of Core-S1. Peak 1 had a retention time of 01:03:08 (hr:min:sec) and corresponds to fraction 6, and peak 2 had a retention time of 01:12:32 and corresponds to fraction 8. The lower panel in the Figure shows the results of Core-S1 detection using anti-pre-S1 monoclonal antibody (antibody 18AN14 at 1/500 dilution) in dot blot analysis of the SEC fractions.
**Figure 6** shows the results of SEC analysis of Core-S1₁₅₄. The retention times of the peaks were as follows:

| Peak | Retention time (hr:min:sec) | Fraction No. |
|---|---|---|
| 1 | 01:03:34 | 6 |
| 2 | 01:11:15 | 7 |
| 3 | 01:17:13 | 8 |
| 4 | 01:59:28 | 17 |
| 5 | 02:16:57 | 20 |

The lower panel in the Figure shows the results of Core-S1₁₅₄ detection using anti-pre-S1 monoclonal antibody (antibody 18AN14 at 1/1000 dilution) in dot blot analysis of the fractions from SEC.

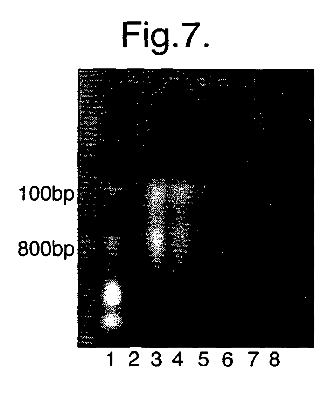

**Figure 7** shows the result of 1.5% agarose gel electrophoresis of DNA purified from Core-S1, Core-S1₁₅₄, Core and Core₁₄₆ immunogens (TBE buffer, 50V for 2h, stained with SYBER I™). Lane 1 = 100bp ladder (1µg), lanes 2 and 5 = blank (empty), lane 3 = Core, lane 4 = Core-S1, lane 6 = Core₁₄₆, lane 7 = Core-S1₁₅₄ and lane 8 = DNA extraction process control. The DNA was extracted from 40 µg of protein.

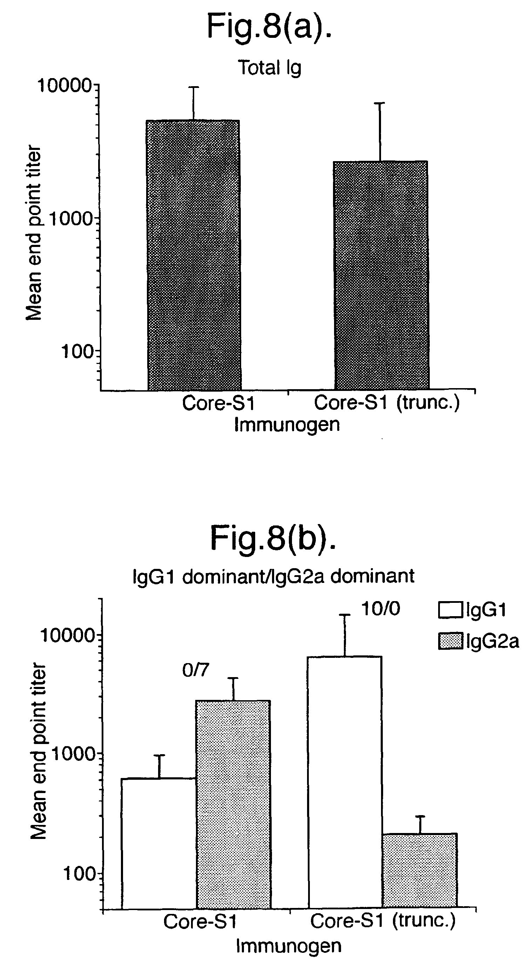

**Figure 8a** shows the total anti-pre-S1 peptide antibody response 14 days after immunisation of mice with Core-S1 or Core-S1₁₅₄. **Figure 8b** shows the anti-pre-S1 peptide IgG subclass response 14 days after immunisation of mice with Core-S1 or Core-S1₁₅₄.

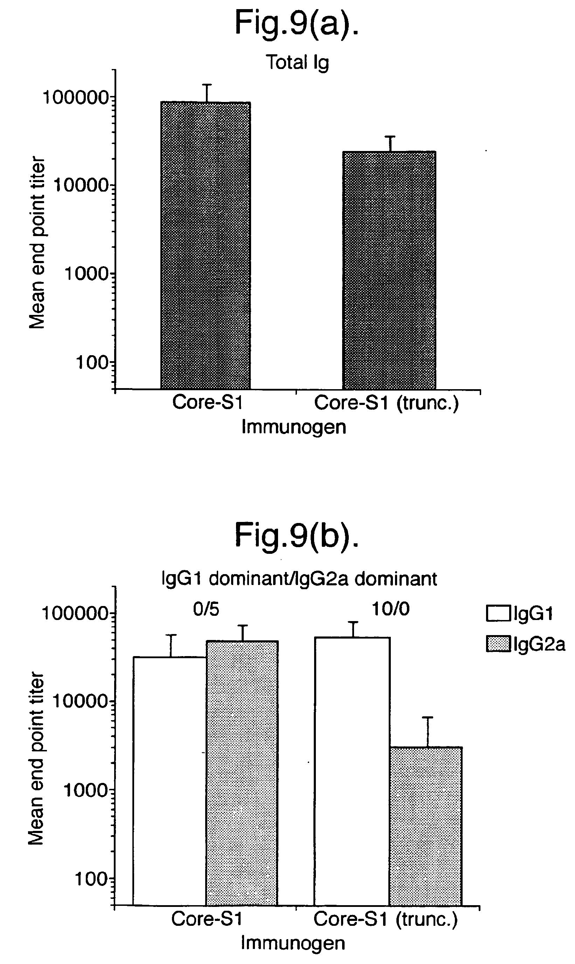

**Figure 9a and 9b** show the same as Figures 8a and 8b respectively, except that the antibody responses were measured at 28 days after immunisation instead of at 14 days.

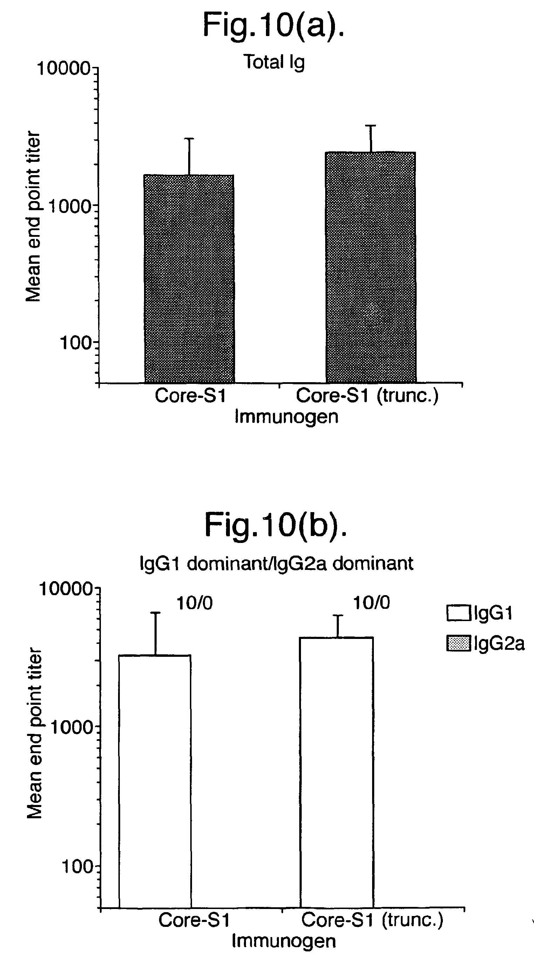

**Figure 10a** shows the total anti-Core₁₄₆ antibody response 14 days after immunisation of mice with Core-S1 or Core-S1₁₅₄. **Figure 10b** shows the anti-Core₁₄₆ IgG subclass response 14 days after immunisation with Core-S1 or Core-S1₁₅₄.

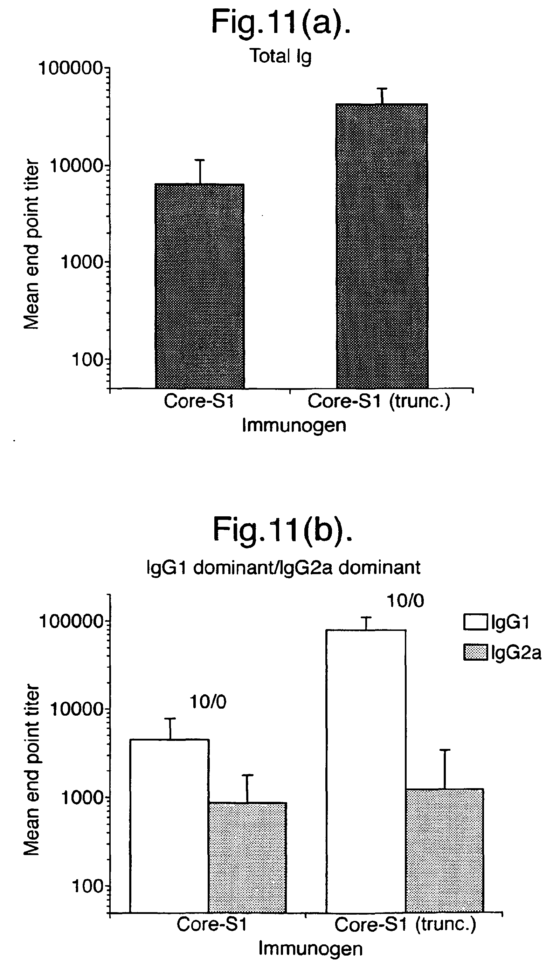

**Figures 11 a and 11b** show the same as Figures 10a and 10b respectively, except that the antibody responses were measured at 28 days after immunisation instead of at 14 days.

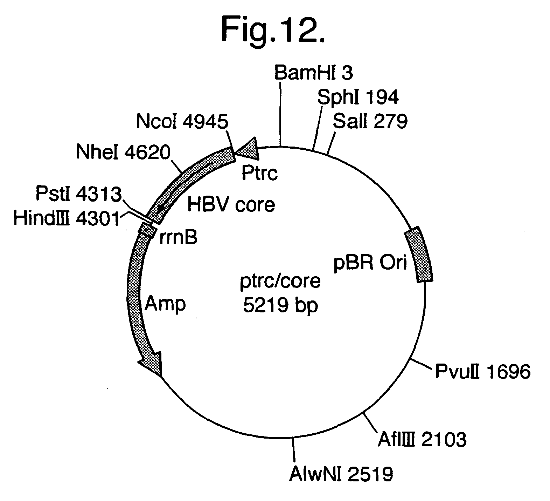

**Figure 12** shows a restriction endonuclease map of plasmid p*trc*/*core.*

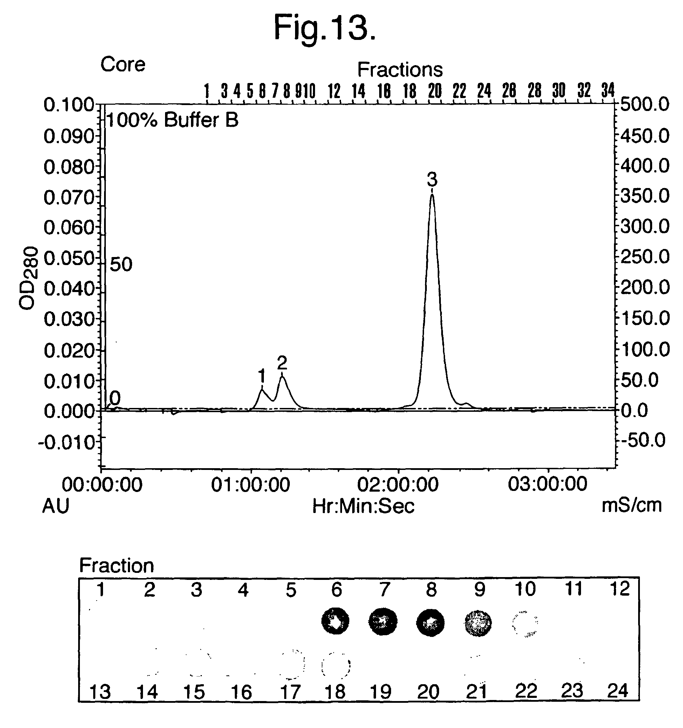

**Figure 13** shows the results of an SEC analysis of Core. The retention times of the peaks were as follows:

| Peak | Retention time | Fraction No. |
|---|---|---|
| | (hr:min:sec) | |
| 1 | 01:04:25 | 6 |
| 2 | 01:12:32 | 8 |
| 3 | 02:13:58 | 20 |

The lower panel in the Figure shows the results of Core detection using anti-Core polyclonal antibody (Dako at 1/1000 dilution) in dot blot analysis of the SEC fractions.

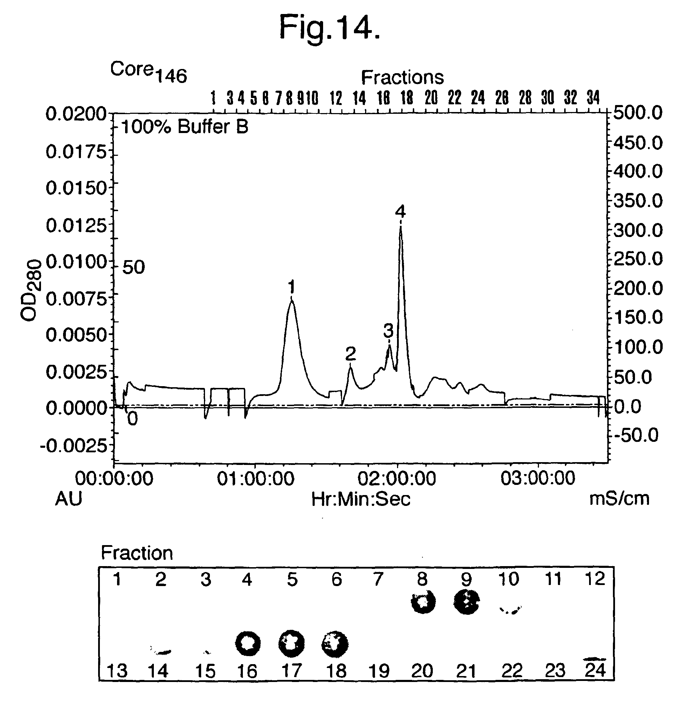

**Figure 14** shows the results of an SEC analysis of Core₁₄₆. The retention times of the peaks were as follows:

| Peak | Retention time (hr:min:sec) | Fraction No. |
|---|---|---|
| 1 | 01:15:31 | 8 |
| 2 | 01:40:28 | 13 |
| 3 | 01:56:41 | 16 |
| 4 | 02:01:48 | 17 |

The lower panel in the Figure shows the results of Core₁₄₆ detection using anti-Core monoclonal antibody (at 1/5 dilution) in dot blot analysis of the SEC fractions.

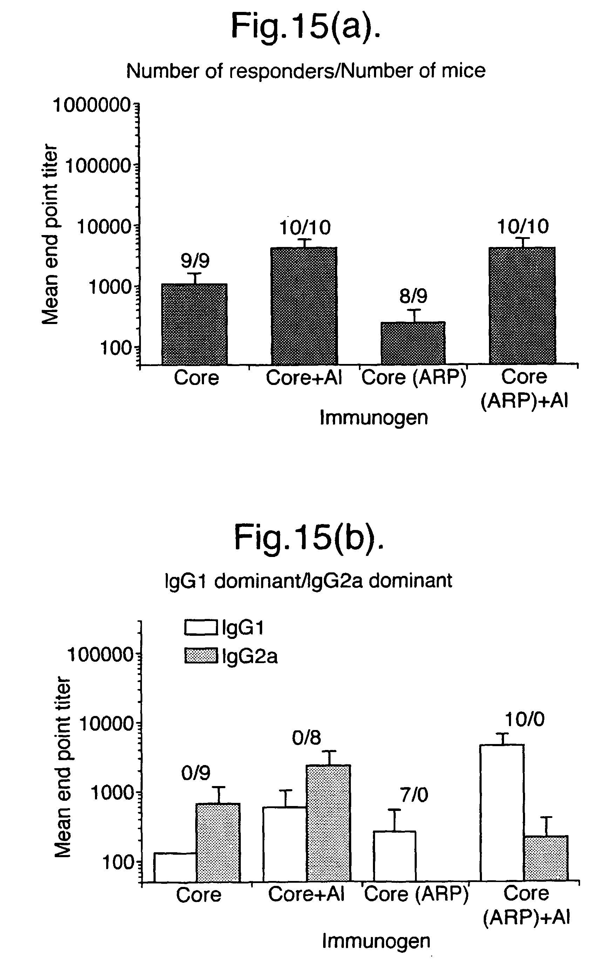

**Figure 15a** shows the total anti-Core₁₄₆ antibody response 14 days after immunisation of mice with Core or Core₁₄₆ ("ARP") in the presence or absence of adjuvant (Alhydrogel, A1). **Figure 15b** shows the anti-Core₁₄₆ IgG subclass response 14 days after immunisation with Core or Core₁₄₆ in the presence or absence of adjuvant.

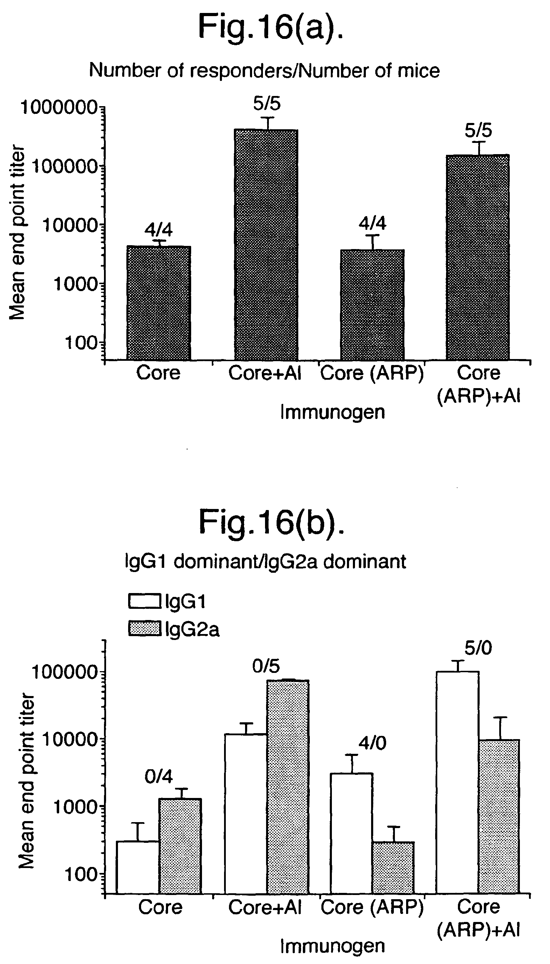

**Figures 16a** and **16b** show the same as Figures 15a and 15b respectively, except that the antibody responses were measured at 42 days after immunisation instead of at 14 days.

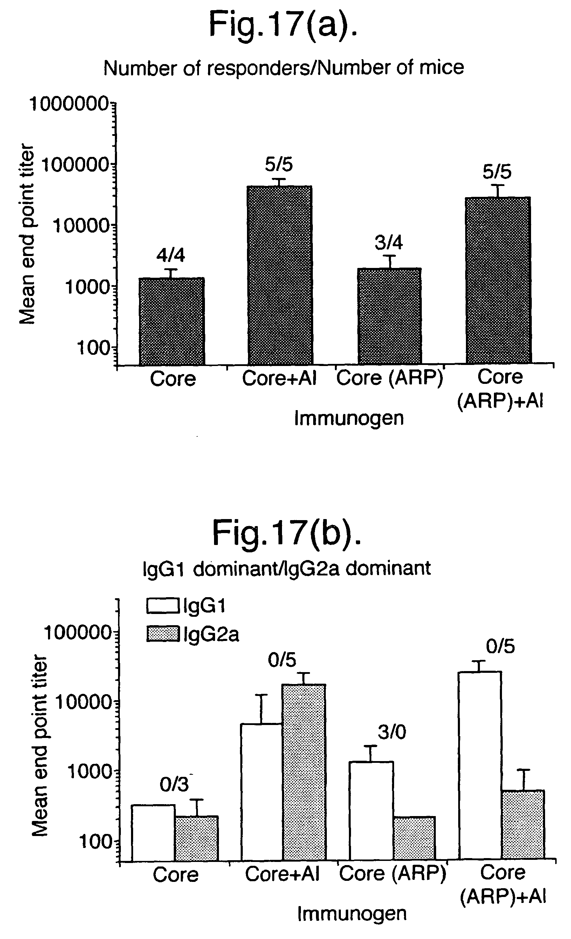

**Figure 17a** shows the total anti-Core-e1 deleted antibody response 42 days after immunisation of mice with Core or Core₁₄₆ in the presence or absence of adjuvant. **Figure 17b** shows the anti-Core-e1 deleted IgG subclass response 42 days after immunisation of mice with Core or Core₁₄₆ in the presence or absence of adjuvant.

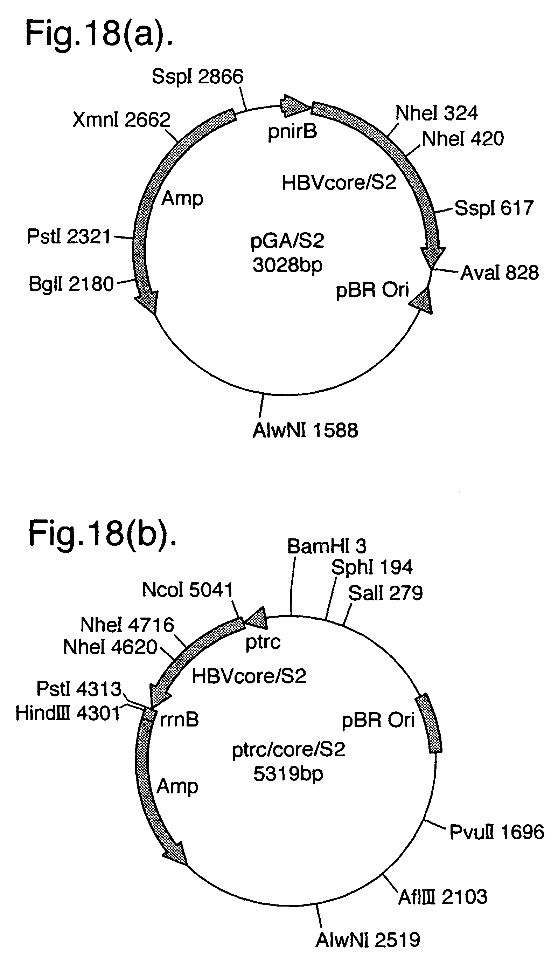

**Figure 18a** is a restriction endonuclease map of plasmid pGA/S2 and **Figure 18b** is a restriction endonuclease map of plasmid ptrc/core/S2.

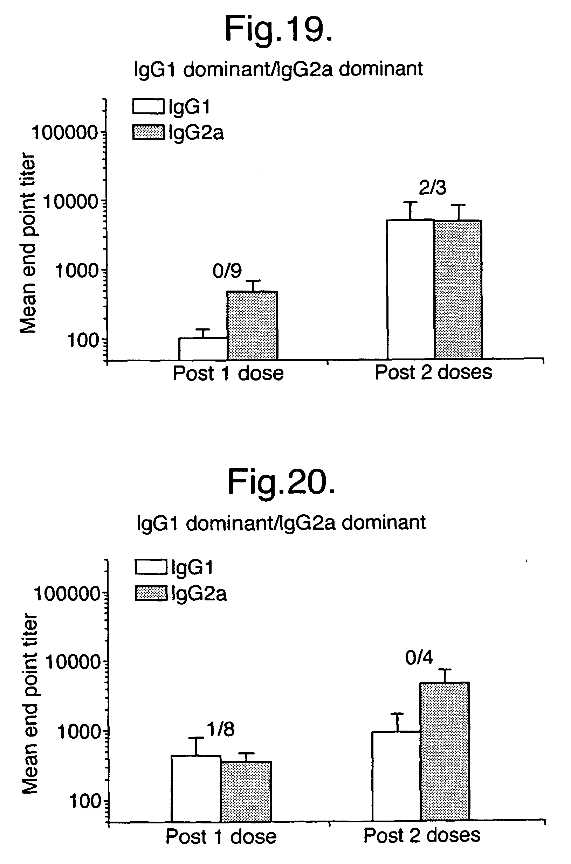

**Figures 19 and 20** show the IgG anti-pre-S2 and anti-Core subclass responses respectively following immunisation with Core containing a pre-S2 insert.

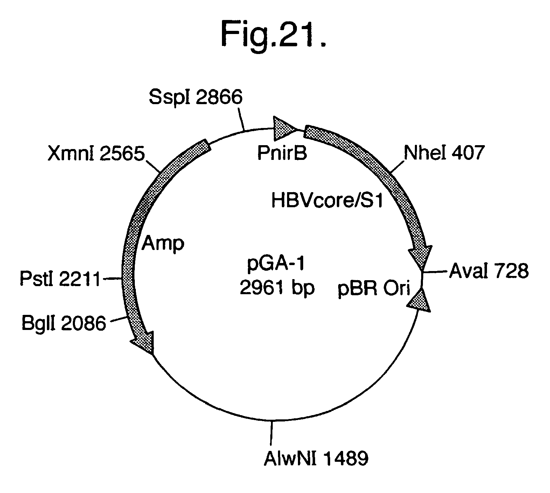

**Figure 21** is a restriction endonuclease map of plamid pGA-1.

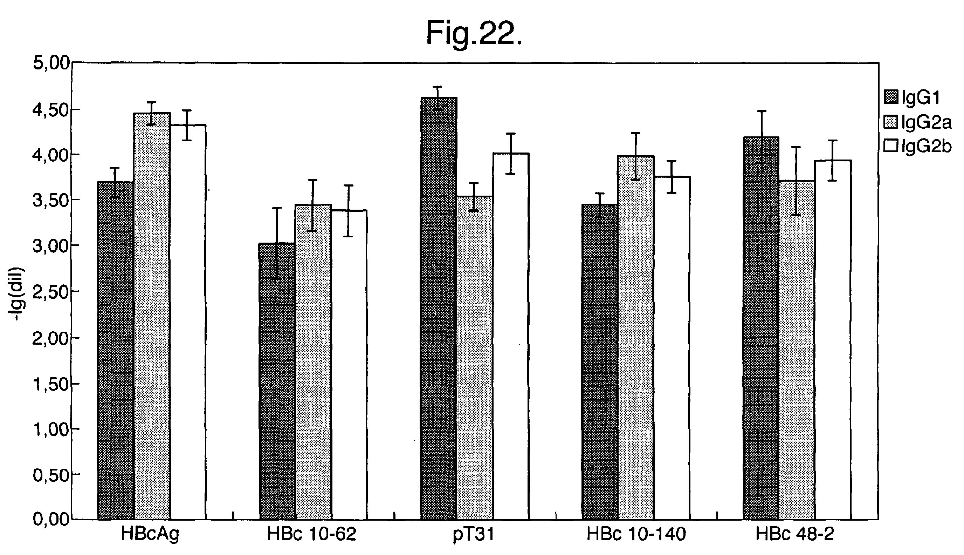

**Figure 22** shows averaged anti-HBc responses of immunised mice. The titres were calculated as the negative logarithms of the EC50 (effective concentration, 50%) serum dilution on the basis of sigmoidal dose-response curves.

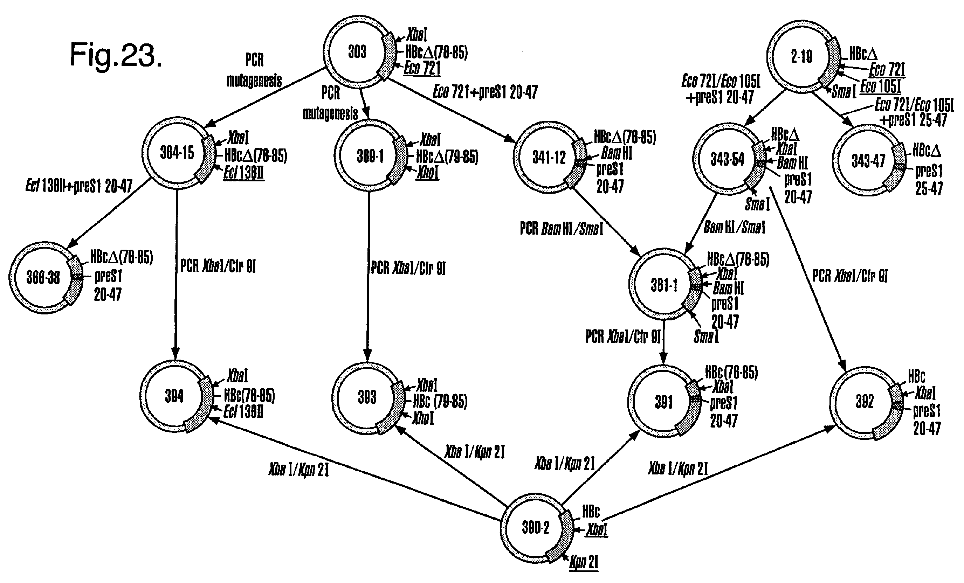

**Figure 23** shows the construction map of HBcΔ-preS1(20-47) derivatives described in Example 6.

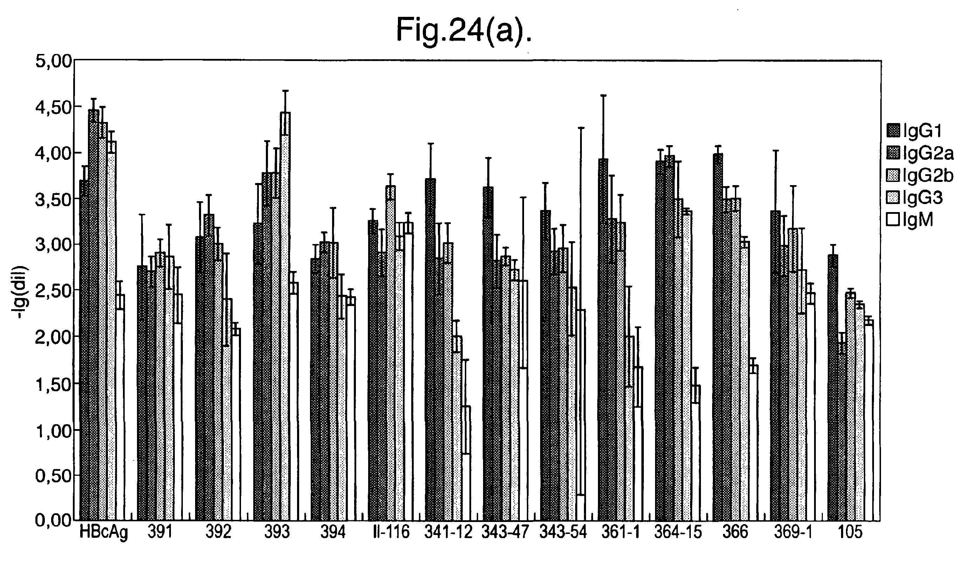

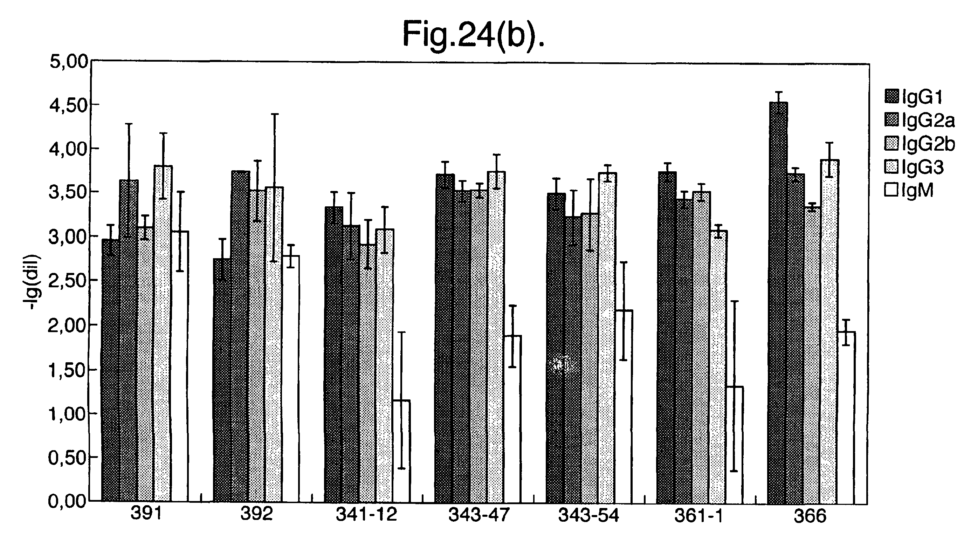

**Figures 24a and 24b** show the averaged anti-HBc and anti-preS1 responses of immunised mice, respectively. The titres were calculated as the negative logarithms of the EC50 (effective concentration, 50%) serum dilution on the basis of sigmoidal dose-response curves.

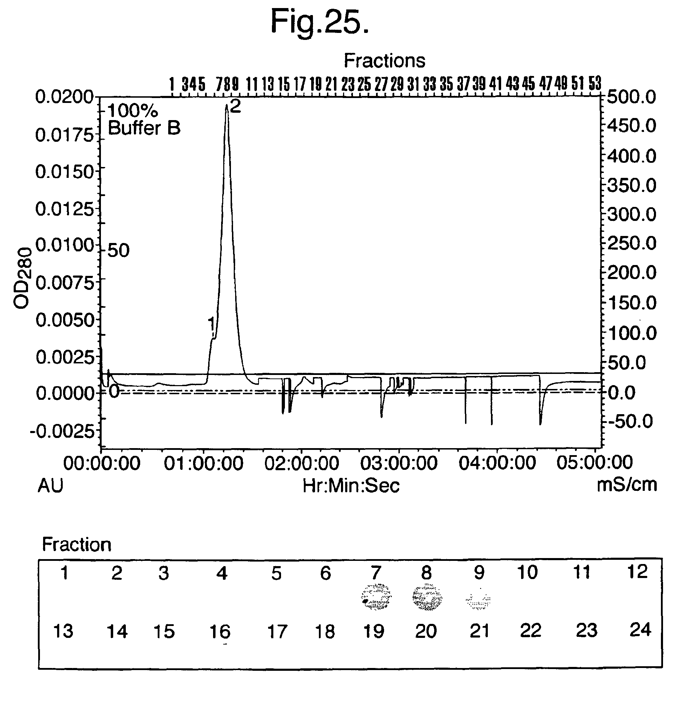

**Figure 25** shows the results of SEC analysis of Core-S1₁₄₆. The retention times of the peaks were as follows:

| Peak | Retention time | Fraction |
|---|---|---|
| 1 | 01:05:16 | 6 |
| 2 | 01:14:40 | 8 |

The lower panel in the Figure shows the results of Core detection using anti-Core monoclonal antibody (at 1/5 dilution) in dot blot analysis of the SEC fractions.

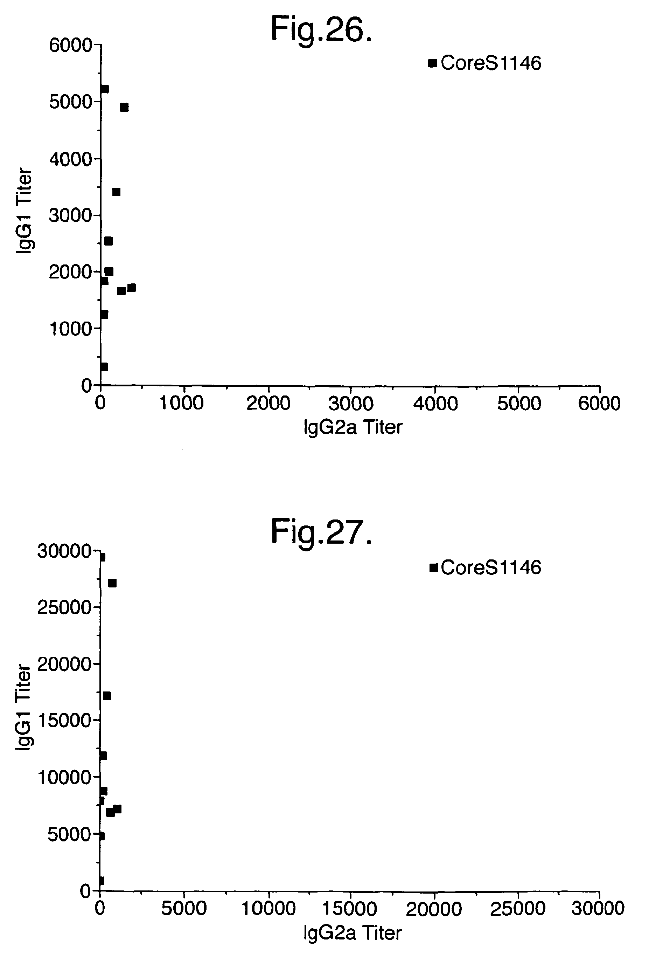

**Figure 26** shows the Ig2a titer against the IgG1 titer in mice at day 14 post one dose of Core-S1₁₄₆.
**Figure 27** shows the Ig2a titer against the IgG1 titer in mice at day 28 post two doses of Core-S1₁₄₆.

### EXAMPLE 1 - Evaluation of the IgG subclass response against an HBV pre-S1 (amino acids 20-47) 'e1'-loop insert, in mice immunised with Core-S1 or Core-S1 truncated at amino acid 154.

### 1 Materials and Methods

### 1.1 Immunogens

### 1.1.1 Core-S1

Core-S1 is a recombinant protein, with the HBV pre-S1 sequence (amino acids 20-47, subtype *ayw*) inserted into the 'e1'-loop of the HBV Core protein sequence between amino acids 79 and 80. The Core protein sequence is 185 amino acids long. Core-S1 was expressed in *Escherichia coli* bacteria (strain HB101), transformed with the plasmid p*trc*/core/S1. This was constructed as follows:

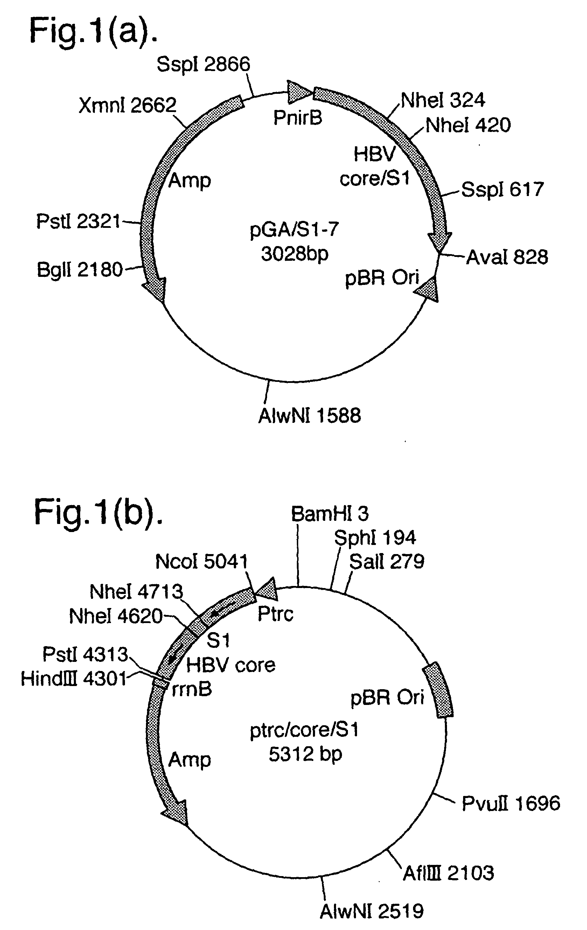

Plasmid pGA/S1-7 (see Figure 1a) was constructed by subcloning a 93 base pair (bp) *Nhe* I polymerase chain reaction (PCR) fragment (which encodes for the pre-S1 amino acid sequence 20-47, subtype *ayw*)), into the *Nhe* I site of pGA-1 (which codes for Core) (see Figure 21). A 728 bp PCR fragment from pGA/S1-7 (which encodes for Core-S1) was digested with *Nco* I/*Pst* I and was subcloned into the *Nco* I/*Pst* I site of the vector pKK233.2 (p*trc,* Pharmacia, Sweden) pre-cut with *Nco* I/*Pst* I, producing the construct ptrc/core/S1, as shown in Figure 1b.

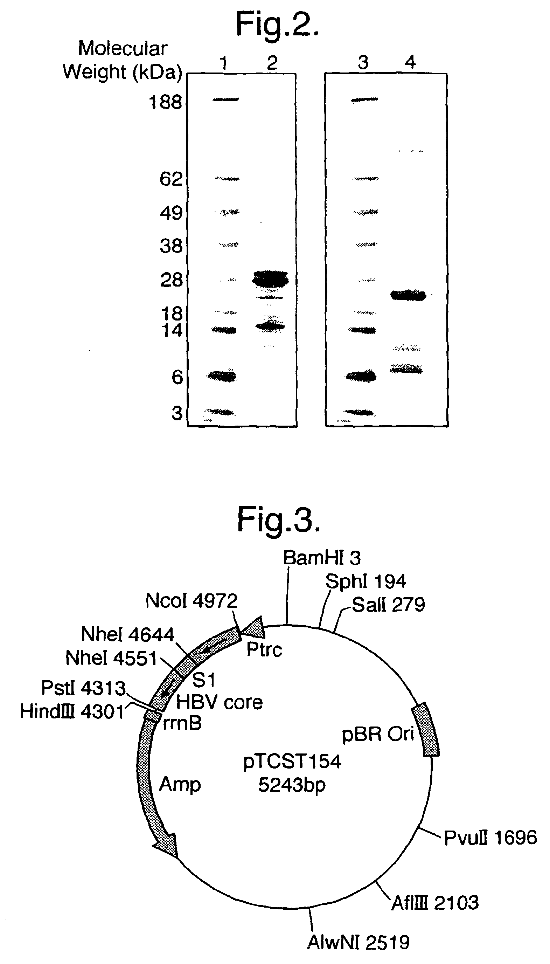

Following lysis of the bacterial cells, Core-S1 particles were purified by buoyant density centrifugation, using sucrose gradients (15 - 45%). They were dialysed against phosphate buffered saline (PBS) (NaCl (0.137M), KCl (2.7 x 10⁻³M), Na₂HPO₄(0.01M), KH₂PO₄(1.5 x 10⁻³M) in H₂O, adjusted to pH = 7.2), for 4 h and then 18h and concentrated using a Microprodicon™ vacuum concentrator (Pierce and Warriner) with a 100 kD molecular weight cut-off membrane. Core-S1 material was quantified by densitometry of Coomassie Colloidal Blue (Sigma)-stained protein separated by reducing sodium dodecyl sulphate (SDS) polyacrylamide gel electrophoresis (PAGE), using Core₁₄₆(see section 1.6.1), as the standard quantity. The purity of the material was > 70% by SDS-PAGE (see Figure 2) and the endotoxin content was 808 endotoxin units (EU)/ml (*Limulus* Amebocyte Lysate (LAL) assay (Kinetic-QCL™-Bio* Whittaker)).

### 1.1.2 Core-S1₁₅₄

Core-S1₁₅₄ is a recombinant protein with the HBV pre-S1 sequence (amino acids 20-47) inserted in the 'e1'-loop of the HBV Core protein sequence (between amino acids 79 and 80), with the Core sequence C-terminally truncated at amino acid position 154. Core-S1₁₅₄ was expressed in *Escherichia coli* bacteria (strain HB101), transformed with the plasmid pTCST₁₅₄. This was constructed as follows:

A 660 bp PCR fragment of ptrc/core/S1 was obtained and digested with *Nco* I/*Pst* I. A 653 bp *Nco* I/*Pst* I fragment, which encodes for Core-S1 (truncated at amino acid 154), was purified and ligated into a 4583 bp *Nco* I/*Pst* I fragment of pKK233.2 (See Figure 3)

Following lysis of the bacterial cells, Core-S1₁₅₄ particles were purified by buoyant density centrifugation, using sucrose gradients (15 - 30%). They were dialysed and concentrated as for Core-S1 particles (see section 1.1.1). Core-S1₁₅₄ material was quantified by densitometry of Coomassie Colloidal Blue (Sigma)-stained protein, separated by reducing SDS-PAGE, using Core₁₄₆ as the standard quantity. The purity of the material was > 70% by SDS-PAGE (see Figure 2) and the endotoxin content was 1182 EU/ml (LAL assay; Kinetic-QCL™ - Bio*Whittaker).

### 1.2 Adjuvant

Alhydrogel adjuvant was used at a final concentration of 2.5mg aluminium hydroxide per ml PBS. Core-S1 or Core-S1₁₅₄ was incubated with Alhydrogel in PBS, at room temperature for 1h to allow adsorption, prior to injection into mice.

### 1.3 Determination of Immunogen Composition

### 1.3.1 Core-S1

Following gradient purification, dialysis and concentration, preparations of Core-S1 were aliquotted into 1.5ml cryo-vials (Nunc™) and frozen to -20°C. An aliquot of this material was thawed to room temperature and used to prepare immunogen stocks. Additionally, a proportion (100µg) was analysed by size exclusion chromatography (SEC). This consisted of a guard column (TSK guard column SWXL™, 6.0mm internal diameter (i.d.) x 4cm; TosoHaas) and a TSK G4000 SWXL™ analytical column (7.8 mm i.d. x 30cm; TosoHaas), attached to a Biologic HR™ system (Biorad). Samples were analysed in PBS buffer (flow rate 0.1ml/min.). Fractions (0.5ml) were collected and maintained at 4°C. Molecular mass standards used for calibration of the column included thyroglobulin (Mw = 669000), Ferritin (Mw = 440000), Catalase (Mw = 232 000) and dextran blue-2000 (derived from the high molecular weight gel filtration calibration kit; Pharmacia).

### 1.3.2 Core-S1₁₅₄

Following gradient purification, dialysis and concentration, preparations of Core-S1₁₅₄ were aliquotted into 1.5ml cryo-vials (Nunc™) and frozen to -20°C. An aliquot of this material was thawed to room temperature and used to prepare immunogen stocks.

Additionally, a proportion (13µg) was analysed using an SEC system (see section 1.3.1). Molecular mass standards were as described for Core-S1 (see section 1.3.1).

### 1.4 Dot Blot Analysis of SEC Fractions

A total of 100 µl of each SEC fraction, for each protein (i.e. Core-S1 or Core-S1₁₅₄), was loaded onto a nitrocellulose filter, using a dot blot chamber. The filter was washed in PBST (PBS + 0.05% Tween 20) for 5 min. and then blocked with 1% bovine serum albumin (BSA) in PBS, for 30 min at room temperature. The filter was washed in PBST for 5 min (x2) at room temperature and then incubated for 1-2h with one of the following (diluted in 0.1% BSA in PBST): anti-pre-S1 monoclonal antibody (18AN14 - gift from Professor W. Gerlich, University of Giessen) at 1/500 (Core-S1) or 1/1000 (Core-S1₁₅₄). Following washing in PBST for 5 min. (x2) at room temperature, the filter was incubated for 1-2h with rabbit anti-mouse (horse radish peroxidase (HRP)-conjugated) polyclonal antibody (Dako Cat. No. P0260) at 1/1000 (both Core-S1 and Core-S1₁₅₄). The filter was washed in PBST for 5 min (x2) and then in PBS for 5 min. (x1), at room temperature. Substrate solution (30mg chloronaphthol dissolved in 10ml methanol, mixed with 30ml H₂O₂ in 50ml PBS) was then incubated with the filter until colour was observed. Finally the reaction was stopped by washing the filter with distilled water.

### 1.5 Determination of DNA Content of Immunogens

### 1.5.1 Core-S1 and Core-S1₁₅₄

Determination of the DNA content of the immunogens was performed using purified material, prior to formulation with Alhydrogel, using a method adapted from Birnbaum and Nasal (1990). Protein (~20 µg) was digested with Proteinase K and the nucleotide was extracted using a commercial DNA recovery kit (Qiagen, QIAquick™ PCR Purification Kit). Purified DNA was visualised using a high sensitivity DNA stain (Novex, SYBER Green I™) in a 1.5% agarose gel, following electrophoresis.

The DNA product obtained following extraction, was quantified using the optical density (OD) 260nm:280nm ratio according to Sambrook *et al.,* (1989) using a Pharmacia Biotech, Ultraspec 2000™.

### 1.6 Antigens

### 1.6.1 HBV Core protein (Core₁₄₆)

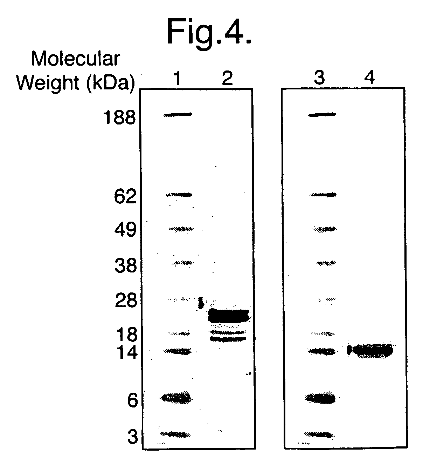

HBV Core protein (Core₁₄₆) was commercially derived from American Research Products (ARP) Inc., Belmont, MA1478, USA (Cat. No. 12-3069; lot No. 21-CIT-0050). The protein contains the 'el'-loop, is C-terminally truncated at amino acid position 146 and derived by expression in recombinant *Escherichia coli* bacteria. The material has a purity of > 90% by SDS-PAGE (see Figure 4) and the endotoxin content was < 24 endotoxin units (EU)/ ml (LAL assay).

### 1.6.2 HBV pre-S1 Peptide

A pre-S1 peptide was derived commercially (GenoSys Biotechnologies, Europe) and had the following amino-acid sequence:
PLGFFPDHQLDPAFRANTANPDWDFNP

This sequence corresponds to residues 21-47 of the surface protein of the HBV pre-S1 protein (subtype *ayw*).

### 1.7 Immunisation Protocol

Two groups of 10 female BALB/c mice (6-8 weeks old) (Harlan) were immunised with 10 µg of either Core-S1 or Core-S1₁₅₄ (adjuvanted with Alhydrogel) by the intraperitoneal (i.p.) route. All immunogens were given in identical volumes (0.5ml) and were administered on days 1 and 14. Five mice per group were sacrificed on day 28 and day 42 and bled out. Sample bleeds were taken on day 14.

### 1.8 Total Ig and IgG Subclass Determination

Serum samples were tested for anti-pre-S1 peptide and anti-Core₁₄₆ antibodies by ELISA. EIA/RIA 96-well microtitre plates (Costar™) were coated with either Core₁₄₆ (0.1 µg well) or pre-S1 peptide (0.1 µg well) in PBS. Sera from individual mice were serially titrated on the test plates, starting at a 1/50 dilution. End-point titres for each sample were determined at the x-axis intercept of the dilution curve at the 0.3 optical density value (with background subtraction). Total Ig antibodies from immunised mice were detected using horse-radish peroxidase conjugated rabbit anti-mouse antibody (Dako Cat. No. P0260, 1/2000 dilution) and IgG subclass specific antibodies were detected using optimally diluted, biotin-labelled rat anti-mouse IgG specific monoclonal antibodies. Dilutions were as follows: IgG1 (1/4000) (Pharmingen Cat. No.: 02232D, Clone: A85-1), IgG2a (1/1000) (Pharmingen, Cat. No. 02012D, Clone R19-15).

### 1.9 Calculation of IgG Subclass Dominance

Serum IgGI :IgG2a titer ratios were calculated for individual mice, for each antigen. If the value was < 0.5, then IgG2a dominance was present. If the ratio was > 2.0, then IgG1 dominance was present. When the value was > 0.5 but < 2.0 then no dominance was present and the response was 'mixed'.

### 1.10 Statistical Analysis

Student's t-test analysis (2 tailed) was performed on the serum antibody data and significance was at the 95% confidence limits (p < 0.05).

### 2. Results

### 2.1 Immunogen Composition

### 2.1.1 Core-S1

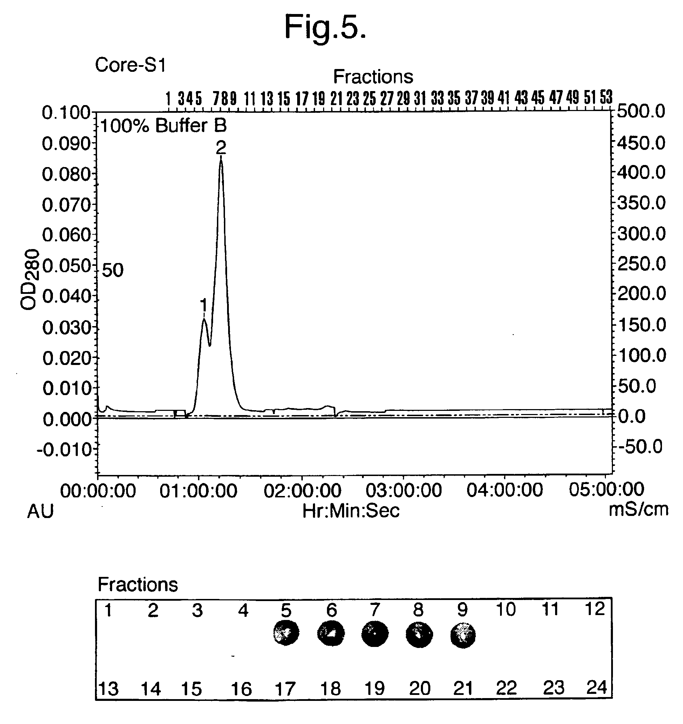

A total of 100 µg of Core-S1 gave the optical density profile shown in Figure 5, when injected into the SEC system. Dot blot analysis using an anti-pre-S1 monoclonal antibody (18AN 14 at 1/500) for detection, showed that all proteins containing the pre-S1 (20-47) epitope were present in fractions 5 to 9 (peak 1 (fraction 6) retention time (r.t.) = 01:03:08; peak 2 (fraction 8) r.t. = 01:12:32), suggesting the presence of a high molecular mass species (most probably particles), based on the molecular mass standards.

### 2.1.2 Core-S1₁₅₄

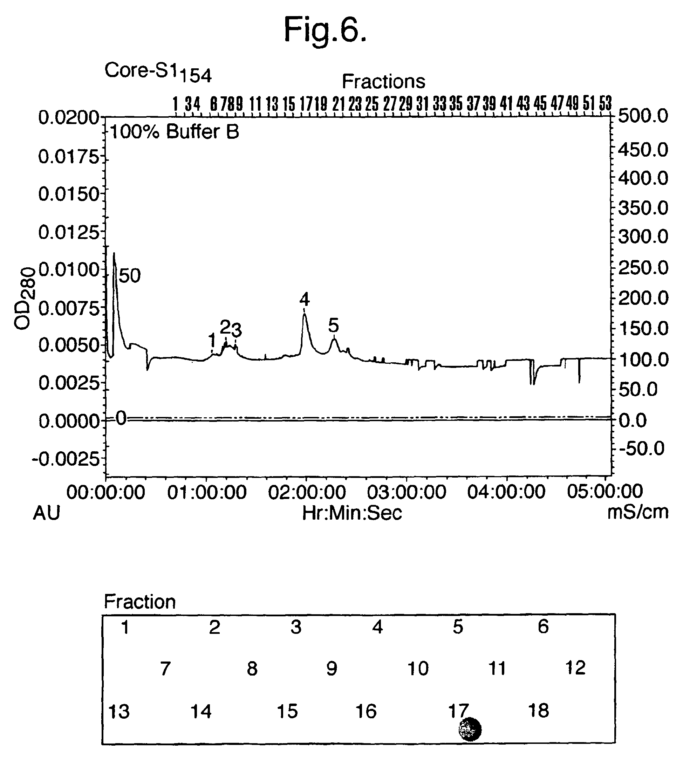

A total of 13 µg of Core-S1₁₅₄ gave the optical density profile shown in Figure 6, when injected into the SEC system. Dot blot analysis using an anti-pre-S1 monoclonal antibody (18AN14 at 1/1000) for detection, showed that proteins containing the pre-S1 (20-47) epitope were present in fractions 7-9 (not apparent from scanned image of nitrocellulose dot blot filter shown in Figure 4; peak 2 (fraction 7) r.t. = 01:11:15; peak 3 (fraction 8) r.t. = O1:17:13) and fraction 17 (peak 4 r.t. = O1:59:28), suggesting the presence of a mixed population of high molecular mass species (probably particles) and lower molecular mass species, based on the molecular mass standards.

### 2.2 DNA Content - Core-S1 and Core-S1₁₅₄

### 2.2.1 Agarose Gel Electrophoresis

DNA was detected in the Core-S1 immunogen preparation (lane 4 of Figure 7) but not in the Core-S1₁₅₄, preparation (lane 7).

### 2.2.2 DNA Evaluation by Spectrophotometry

DNA was recovered from the Core-S1 immunogen only (see Table 1), thus correlating with the agarose gel electrophoresis results. The DNA values were 14 ng DNA/µg protein (stdev = 2) (Core-S1) and <3 ng DNA/µg protein (Core-S1₁₅₄).

**Table 1**

| Immunogen | DNA yield (ng DNA/µg protein)^{a} | St. dev. (n=5) |
|---|---|---|
| Core - S1 | 14 | 2 |
| Core - S1₁₅₄ | <3 | - |

| | | |
|---|---|---|
| "Ref :- Sambrook *et al.,* 1989. | | |

### 2.3 Induction of Anti-pre-S1 Peptide Antibodies

Anti-pre-S1 peptide Ig responses were detected in mice immunised with either Core-S1 or Core-S1₁₅₄, at day 14 (post 1 dose) (see Figure 8a). There was no significant difference in the titers (p = 0.132). At day 28 (post 2 doses) the Ig response against pre-S peptide was significantly higher for mice immunised with Core-S1 (p = 0.002) (see Figure 9a).

### 2.4 Induction of Anti-Core₁₄₆ Antibodies

Anti-Core₁₄₆ Ig responses were not significantly different at day 14 (post 1 dose) (p = 0.225), in mice immunised with either Core-S1 or Core-S1₁₅₄ (see Figure 10). At day 28 (post 2 doses), mice immunised with Core-S1₁₅₄ showed significantly higher anti-Core₁₄₆ responses (p < 0.001) (see Figure 11a).

### 2.5 Anti-pre-S1 Peptide IgG Subclass Responses

The mean anti-pre-S1 peptide (amino acids 21-47) IgG2a titer was significantly higher (p = 0.003) in mice immunised with Core-S1, whereas the mean anti-pre-S1 peptide IgG1 response was significantly higher (p = 0.04) in the Core-S1₁₅₄ group, at day 14 (see Figure 8b). In the Core-S1 group, 7/10 mice showed an individual anti-pre-S1 peptide IgG1:IgG2a titer ratio of < 0.5, suggesting IgG2a dominance. In the Core-S1₁₅₄ group 10/10 mice showed an IgG1 dominance (individual IgG1:IgG2a titer ratio of > 2.0).

At day 28, the Core-S1 group showed a significantly higher mean anti-pre-S1 IgG2a titer (p < 0.001) but the Core-S1₁₅₄ group showed a significantly higher mean anti-pre-S1 IgG1 response (p = 0.017) (see Figure 9b). Five mice in the Core-S1 group showed IgG2a dominance, whereas 10/10 mice showed IgG1 dominance in the Core-S1₁₅₄ group.

### 2.6 Anti-Core₁₄₆ Subclass Responses

At 14 days (post 1 dose) anti-Core₁₄₆ IgG2a responses were not detected in mice immunised with either the Core-S1 or Core-S1₁₅₄ and the anti-Core₁₄₆ IgG1 responses were not significantly different (p = 0.393) (see Figure 10b). A total of 10/10 mice showed an IgGI dominance in either group. Mean anti-Core₁₄₆ IgGI titers were significantly higher (p < 0.001) in the Core-S1₁₅₄ group at 28 days (see Figure 11b).

### 3.1 Relationship Between Immunogen Structure and Ig response

The Core-S1 immunogen consists exclusively of a single, pre-S1 epitope containing, high molecular mass species (probably particles) and induces a dominant IgG2a (Th1 type) response against the pre-S1 (amino acids 20-47) sequence, inserted into the 'e1'-loop. Higher anti-pre-S1 peptide total Ig responses were also induced, compared with Core-S1₁₅₄.

The Core-S1₁₅₄ immunogen contains two populations of proteins (high and low molecular mass), containing the pre-S1 (amino acids 20-47) epitope. A dominant IgG1 (Th2 type) response against the pre-S1 (amino acids 20-47) sequence and a higher total anti-Core₁₄₆ Ig response were induced.

### 4. Endotoxin

Endotoxin is documented to influence the response towards a Th1 response. However, the endotoxin content of Core-S1₁₅₄ (1182 EU/ml) was found to be higher than Core-S1 (808 EU/ml), but the former induced a Th2 dominated response against the e1 loop insert. This suggests that endotoxin is not responsible for the switch.

### EXAMPLE 2 - Evaluation of the IgG subclass response against HBV Core protein 'e1'-loop in mice immunised with HBV Core or HBV Core truncated at amino acid 146.

### 1 Materials and Methods

### 1.1 Immunogens

### 1.1.1 HBV Core protein (Core)

HBV Core protein (amino acids 1-185) was expressed in *Escherichia coli* bacteria (strain HB 101) transformed with the plasmid p*trc*/Core. This was constructed as follows:

A 636 bp *Nco* I/*Pst* I digested, pGA-1 (Fig. 21) PCR fragment (which encodes for the full length Core protein) was subcloned into a 4583 bp *Nco* I*lPst* I fragment of pKK233.2 (p*trc*, Pharmacia, Sweden). This generated a ptrc/Core plasmid, which expressed HBV Core under the *trc* promoter (see Figure 12).

Following lysis of the bacterial cells, Core particles were purified by buoyant density centrifugation, using sucrose gradients (15 - 45%). They were dialysed against PBS for 4h and then 18h and concentrated using Aquacide™ (Calbiochem Cat. No. 17851). Core protein was quantified by densitometry of Coomassie Colloidal Blue (Sigma)-stained protein, separated by reducing sodium dodecyl sulphate (SDS) polyacrylamide gel electrophoresis (PAGE), using Core₁₄₆ as the standard quantity. The purity of the material was > 80% by SDS-PAGE (see Figure 4) and the endotoxin content was 800 EU/ ml (*Limulus* Amebocyte Lysate (LAL) assay (Kinetic-QCL™ - Bio*Whittaker).

### 1.1.2 HBV Core protein (Core₁₄₆)

HBV Core protein (Core₁₄₆) was commercially derived from American Research Products (ARP) Inc., Belmont, MA1478, USA (Cat. No. 12-3069; lot No. 21-CIT-0050). The protein contains the 'el'-loop, is C-terminally truncated at amino acid position 146 and derived by expression in recombinant *Escherichia coli* bacteria. The material is stated to have a purity of > 90% by SDS-PAGE (see Figure 4) and the endotoxin content was < 24 EU/ ml (LAL assay; Kinetic-QCL™ - Bio*Whittaker).

### 1.2 Adjuvant

Alhydrogel adjuvant was used at a final concentration of 1.44 mg/ml aluminium hydroxide in PBS. Core or Core₁₄₆ protein was incubated with Alhydrogel in PBS at room temperature for 1h to allow adsorption, prior to injection into mice.

### 1.3 Determination of Immunogen Composition

### 1.3.1 Core

Following gradient purification, dialysis and concentration, preparations of Core were aliquotted into 1.5ml cryo-vials (Nunc™) and frozen to -20°C. An aliquot of this material was thawed to room temperature and used to prepare immunogen stocks. Additionally, a proportion (20 µg) was analysed by size exclusion chromatography (SEC). This consisted of a guard column (TSK guard column SWXL™, 6.0mm internal diameter (i.d.) x 4cm; TosoHaas) and a TSK G4000 SWXL™ analytical column (7.8mm i.d. x 30cm; TosoHaas), attached to a Biologic HR™ system (Biorad).

Samples were analysed in PBS buffer (flow rate 0.1ml/ min.). Fractions (0.5ml) were collected and maintained at 4°C. Molecular mass standards used for calibration of the column included thyroglobulin (Mw = 669000), Ferritin (Mw = 440000), Catalase (Mw = 232 000) and dextran blue-2000 (derived from the high molecular weight gel filtration calibration kit; Pharmacia).

### 1.3.2 Core₁₄₆

Core₁₄₆ was aliquotted into Eppendorf™ tubes and frozen to -20°C. An aliquot was thawed to room temperature and used to prepare immunogen stocks. Additionally, a proportion (40 µg) was analysed using an SEC system (see section 1.3.1). Molecular mass standards were as described for Core (section 1.3.1).

### 1.4 Dot Blot Analysis of SEC Fractions

A total of 100 µl of each SEC fraction, for each protein (i.e. Core or Core₁₄₆) was loaded onto a nitrocellulose filter, using a dot blot chamber. The filter was washed in PBST (PBS + 0.05% Tween 20) for 5 min. and then blocked with 1% Bovine Serum Albumin (BSA) in PBS, for 30 min at room temperature. The filter was washed in PBST for 5 min. (x2) at room temperature and then incubated for 1-2h with one of the following (diluted in 0.1% BSA in PBST): anti-Core polyclonal antibody (Dako Cat. No. 0586) at 1/1000 (Core), or anti-Core monoclonal antibody at 1/5 (Core₁₄₆). Following washing in PBST for 5 min (x2) at room temperature, the filter was incubated for 1-2h with either goat anti-rabbit (Horse Radish Peroxidase (HRP)-conjugated) polyclonal antibody (Biorad Cat. No. 172-1013) at 1/1000 (Core), or rabbit anti-mouse-HRP polyclonal antibody (Dako Cat. No. P0260) at 1/1000 (Core₁₄₆). The filter was washed in PBST for 5 min (x2) and then in PBS for 5 min (x1), at room temperature. Substrate solution (30mg chloronaphthol dissolved in 10ml methanol, mixed with 30ml H₂O₂ in 50ml PBS) was then incubated with the filter until colour was observed. Finally the reaction was stopped by washing the filter with distilled water.

### 1.5 Determination of DNA Content of Immunogens

### 1.5.1 Core and Core₁₄₆

Determination of the DNA content was performed on purified immunogens, prior to formulation with Alhydrogel, using a method adapted from Birnbaum and Nasal (1990). Protein (~40 µg) was digested with Proteinase K and the nucleotide extracted using a commercial DNA recovery kit (Qiagen, QIAquick™ PCR Purifiaction Kit). Purified DNA was visualised by a high sensitivity DNA stain (Novex, SYBER Green I™) in a 1.5% agarose gel, following electrophoresis. The DNA product obtained following extraction, was quantified using the optical density (OD) 260nm: 280nm ratio according to Sambrook *et al.,* (1989) using a Pharmacia Biotech, Ultraspec 2000™.

### 1.6 Antigens

### 1.6.1 HBV Core protein (Core₁₄₆)

See Section 1.1.2.

### 1.6.2 HBV Core protein with the 'e1'-loop sequence removed (Core-e1 deleted)

This protein contains a deletion of the Core amino acids between residues 79 and 93. It was derived by expression in recombinant *Escherichia coli* bacteria (strain K802) and purified by SEC. The material has a purity of > 90% by SDS-PAGE.

### 1.7 Immunisation Protocol

Four groups of 10 female BALB/c mice (6-8 weeks old) (Harlan) were immunised with 10 µg of either Core or Core₁₄₆ (+/- Alhydrogel adjuvant) by the i.p. route. All immunogens were given in identical volumes administered on days 1 and 14. Five mice per group were sacrificed on day 28 and day 42 and bled out. Sample bleeds were taken on day 14.

### 1.8 Total Ig and IgG Subclass Determination

Serum samples were tested for anti-Core₁₄₆ or anti-Core-e1 deleted antibodies by ELISA. EIA/RIA 96-well microtitre plates (Costar™) were coated with either Core₁₄₆ (0.1 µg/well) or Core-e1 deleted (1 µg/ well) in PBS. Sera from individual mice were serially titrated on the test plates, starting at a 1/50 dilution. End-point titres for each sample were determined at the x-axis intercept of the dilution curve at the 0.3 optical density value (with background subtraction). Total Ig antibodies from immunised mice were detected using horse-radish peroxidase conjugated rabbit anti-mouse antibody (Dako Cat. No. P0260, 1/2000 dilution) and IgG subclass specific antibodies were detected using optimally diluted, biotin-labelled rat anti-mouse IgG specific monoclonal antibodies. Dilutions were as follows: IgGI (1/4000) (Pharmingen Cat. No.: 02232D, Clone : A85-1), IgG2a (1/1000) (Pharmingen, Cat. No. 02012D, Clone : R19-15).

### 1.9 Calculation of IgG Subclass Dominance

Serum IgG1:IgG2a titer ratios were calculated for individual mice, for each antigen. If the value was < 0.5, then IgG2a dominance was present. If the ratio was > 2.0, then IgG1 dominance was present. When the value was > 0.5 but < 2.0 then no dominance was present and the response was classified as 'mixed'.

### 1.10 Statistical Analysis

Student's t-test analysis (2 tailed) was performed on the serum antibody data and significance was at the 95% confidence limits (p < 0.05).

### 2 Results

### 2.1 Immunogen Composition

### 2.1.1 Core

A total of 20 µg of Core gave the optical density profile shown in Figure 13, when injected into the SEC system. Dot blot analysis using an anti-Core polyclonal antibody (Dako Cat. No. 0586 at 1/1000) for detection, showed that all proteins containing Core epitopes were present in fractions 6 to 10 (peak 1 (fraction 6) retention time (r.t.) = 01.04:25; peak 2 (fraction 8) r.t. = 01:12:32)), suggesting the presence of a high molecular mass species (most probably particles), based on the molecular mass standards. (Peak 3, fraction 20, is due to Aquacide™ - used to concentrate the Core material).

### 2.1.2 Core₁₄₆

A total of 40 µg of Core₁₄₆ showed the optical density profile shown in Figure 14, when injected into the SEC system. The dot blot profile of the Core₁₄₆ material showed that Core epitope containing proteins (assessed using an anti-Core monoclonal antibody at 1/5 for detection) were present in fractions 8-10 (peak 1 (fraction 8) retention time (r.t.) = 01:15:31) and 16-18 (peak 3 (fraction 16) retention time (r.t.) = 01:56:41; peak 4 (fraction 17) r.t. = 02:01:48)), suggesting the presence of a mixed population of high molecular mass species (probably particles) and lower molecular mass species, based on the molecular mass standards.

### 2.2 DNA Content - Core and Core₁₄₆

### 2.2.1 Agarose Gel Electrophoresis

DNA was only detected in the Core immunogen preparation (lane 3 of Figure 7). There was no detectable DNA in the Core₁₄₆ preparation (lane 6).

### 2.2.2 DNA Evaluation by Spectrophotometry

Spectrophotometric results (see Table 2) confirmed the results obtained from the agarose gel analysis, i.e. DNA was only detected in the Core immunogen preparation (24 ng/µg protein, stdev = 4).

**Table 2**

| Immunogen | DNA yield (ng/mg protein)^{a} | St. dev. (n=5) |
|---|---|---|
| Core | 24 | 4 |
| Core₁₄₆ | <3 | - |

| | | |
|---|---|---|
| ^{a}Ref :- Sambrook *et al.,* 1989. | | |

### 2.3 Induction of Anti-Core₁₄₆ Antibodies

Anti-Core₁₄₆ (containing the 'el'-loop) Ig responses were detected in mice immunised with either Core or Core₁₄₆ (+/- Alhydrogel), at day 14 (post 1 dose) (see Figure 15a). Mice immunised with Alhydrogel adjuvanted material showed significantly higher anti-Core₁₄₆ responses at either day 14 (Core p < 0.001; Core₁₄₆ p < 0.001) (see Figure 15a) or day 42 (Core p = 0.034; Core₁₄₆ p = 0.023) (see Figure 16a). There was no significant difference in the anti-Core₁₄₆ response between mice immunised with adjuvanted Core or Core₁₄₆, at either day 14 (p = 0.78) or 42 (p = 0.05) (see Figures 15a and 16a respectively).

### 2.4 Induction of Anti-Core e1-Deleted Antibodies

Anti-Core e1-deleted Ig responses were detected in mice immunised with either Core or Core₁₄₆ (+/- Alhydrogel adjuvant), at day 42 (see Figure 17a). There was no significant difference between the responses in mice immunised with adjuvanted Core or Core₁₄₆ at day 42 (p = 0.106). (Serum samples taken at day 14 were not tested.)

### 2.5 Anti-Core₁₄₆ IgG Subclass Responses

Anti Core₁₄₆ IgG2a responses were significantly higher (p = 0.008) in mice immunised with Core (+ Alhydrogel) compared with Core₁₄₆ (+ Alhydrogel), at day 14 (see Figure 15b). However, anti-Core₁₄₆ IgGI responses were significantly higher in mice immunised with Core₁₄₆ (+ Alhydrogel) (p < 0.001). Similar trends were noted in mice immunised with non-adjuvanted Core or Core₁₄₆. In the group immunised with Core (+ Alhydrogel), 8/10 mice showed an individual anti-Core₁₄₆ IgG1:IgG2a ratio of < 0.5, suggesting IgG2a dominance. In the group immunised with Core₁₄₆ (+ Alhydrogel), 10/10 mice showed an individual IgGI :IgG2a ratio of > 2.0, suggesting IgGI dominance. Similar trends were noted in mice immunised with non-adjuvanted Core or Core₁₄₆.

At 42 days, mice immunised with Core (+ Alhydrogel) showed a significantly higher mean anti-Core IgG2a titer than mice immunised with Core₁₄₆ (+ Alhydrogel) (p < 0.001) (see Figure 16b). Mean anti-Core₁₄₆ IgG1 titers were significantly higher in mice immunised with Core₁₄₆ (+ Alhydrogel) than in mice immunised with Core (+ Alhydrogel) (p = 0.002). A total of 5/5 mice in the Core (+ Alhydrogel) group showed IgG2a dominance, whereas 5/5 mice in the Core₁₄₆ (+ Alhydrogel) group, showed IgG1 dominance. Similar trends were noted in mice immunised with non-adjuvanted materials.

### 2.6 Anti-Core e1-Deleted IgG Subclass Responses

At 42 days, mean anti-Core e1-deleted IgG2a titers were significantly higher in mice immunised with Core (+ Alhydrogel) than in mice immunised with Core₁₄₆ (+ Alhydrogel) (p = 0.0025) (see Figure 17b). Anti-Core e1-deleted IgG1 responses were significantly higher in mice immunised with Core₁₄₆ (+ Alhydrogel) than in mice immunised with Core (+ Alhydrogel) (p = 0.005). In the Core (+ Alhydrogel) immunised group, 5/5 mice showed IgG2a dominance whereas in the Core₁₄₆ (+ Alhydrogel) immunised group, 5/5 mice showed IgG1 dominance.

### 3 Relationship Between Immunogen Structure and Ig Response

The Core immunogen consists exclusively of a single high molecular mass species (probably particles) and induces a dominant IgG2a (Th1 type) response against the 'e1'-loop and non-'e1'-loop antibody epitopes of HBV Core antigen.

The Core₁₄₆ contains two populations of proteins (high and low molecular mass) containing a Core antibody epitope. A dominant IgG1 (Th2 type) response against 'e1'-loop and non-'e1'-loop antibody epitopes of HBV Core antigen was induced.

### EXAMPLE 3 - Properties of Core-SI₁₄₆, an HBc derivative with a pre-S1 insert in the e1 loop and a truncation at position 146

The Core-S1₁₄₆ product was provided by a collaborator of the inventors, Dr Paul Pumpens of the University of Latvia. The product was analysed by the inventors to determine whether it has a particulate structure and whether it induces a Th1 or Th2 dominated response using the methods described above in Examples 1 and 2.

The Core-S1₁₄₆ was found to be exclusively particulate (Figure 25) and to induce an IgG1 (Th2) dominated response against the pre-S1 insert at both day 14 post one dose (Figure 26) and at day 28 post two doses (Figure 27). This indicates that the presence of a particulate structure by itself is not responsible for inducing a Th1 response.

### EXAMPLE 4 - Evaluation of IgG subclass response against Core having an HBV pre-S2 (amino acids 139-174) 'e1'- loop insert

### 1. MATERIALS AND METHODS

### 1.1 IMMUNOGENS

Hepacore PS2 is a recombinant full length hybrid HBcAg containing the pre-S2 sequence of HBsAg (amino acids 139-174) inserted into the e1 loop between amino acids 79 and 80. Hepacore PS2 was purified by sucrose density gradient centrifugation following expression from *E. coli* bacteria (HB101 strain) transformed with the plasmid *ptrc*/core/S2. This was constructed as follows:

Plasmid pGA/S2 (a schematic representation is shown in Figure 18a) was constructed by subcloning a 96bp *Nhe* I PCR fragment which encodes pre-S2 (139-174 amino acids, *ayw* subtype) into the *Nhe* I site of pGA- 1 (a plasmid which encodes core) (see Figure 21). A 728bp PCR fragment from pGA/S2 (which encodes core-S2) was digested with *Nco* I/*Pst* I and was subcloned into the *Nco* I/*Pst* I site of the vector pKK233-2 (p*trc*, Pharmacia, Sweden) precut with *Nco* I*lPst* I producing the construct p*trc*/core-S2 as represented in Figure 18b.

Alhydrogel adjuvant was used at a final concentration of 1.44mg/ml aluminium hydroxide in phosphate buffered saline (NaCl (0.137M), KCl (2.7 x 10⁻³M) Na₂HPO4 (O.O1M), KH₂PO₄ (1.5 x 10⁻³M), in H₂O adjusted to pH = 7.2).

### 1.2 IMMUNISATION PROTOCOL

A group of ten female BALB/c mice was immunised intraperitoneally with Hepacore PS2 (II) with Alhydrogel adjuvant. Mice were immunised on day 1 with 20 µg and on day 24 with 10 µg of Hepacore PS2 (II). Another group (group B) was immunised on day 1 with 10 µg and on day 24 with 2.5 µg. The mice were bled on days 14 and 49 for antibody analysis.

### 1.3 ANTIGENS USED IN ELISA

Truncated hepatitis B core particles (Core₁₄₆) were commercially derived from American Research Products Inc, Belmont, MA1478, USA (cat. No. 12-3069; lot No. 21-CIT-0050) as described above.

Qβ-S2 recombinant particles were expressed in *E.coli* bacteria and are Qβ bacteriophages expressing the entire pre-S2 sequence of HBsAg (*ayw* subtype). Qβ particles without pre-S2 expression were also used as negative controls in antibody ELISA assays.

### 1.4 IgG SUBCLASS DETERMINATION

IgG subclass determination was carried out as in Example 1, except that the microtitre plates were coated with either Qβ-S2 particles (10µg/ml, 100µl/well) or Core₁₄₆ antigen (1 µg/ml, 100µl/well) to allow determination of anti-pre-S2 antibodies and anti-hepatitis B core antibodies.

### 2. RESULTS

### 2.1 INDUCTION OF ANTI-PRE-S2 ANTIBODIES

High titre anti-pre-S2 antibodies were generated in mice immunised with Hepacore PS2 (II) (Figure 19).

### 2.2 INDUCTION OF ANTI-CORE ANTIBODIES

Anti-core antibodies were generated (Figure 20) in mice immunised with Hepacore PS2 (II).

### 2.3 IGG SUBCLASS RESPONSES

The IgG response to the pre-S2 insert was predominantly of the IgG2a subclass but was more noticeable after one than two immunisations. The IgG subclass response to Core₁₄₆ was also predominantly of the IgG2a subclass.

### EXAMPLE 5 - Expression and properties of HBc derivatives with the C-terminal sequence replaced by long foreign insertions

The experiments described in this Example were carried out in the laboratory of a collaborator of the inventors, Dr Paul Pumpens.

### Summary

Full-length and C-terminally truncated hepatitis B core antigen (HBc) derivatives, which carried long foreign amino acid insertions at position 144, were constructed. HBV pres 1, preS2, and HIV-1 Gag fragments of 50-100 amino acids in length were used as such insertions, and the appropriate recombinant genes were expressed in *E.coli* cells. The appropriate chimeric HBc and HBcΔ derivatives were purified and examined antigenically and immunogenically. Subclass analysis of the induced anti-HBc immune response in mice showed that the Ig ratio of IgG1, IgG2a, and IgG2b antibodies was restored from the IgG1>IgG2a≥IgG2b pattern, which is typical for C-terminally truncated HBcΔ derivatives, to IgG2a≥IgG2b≥IgG1, which is typical for full-length HBc derivatives, after immunisation with C-terminally truncated HBcΔ derivatives, which carried long C-terminal additions of 50-100 amino acids in length.

### Materials and Methods

### Bacterial Strains

*E.coli* strains RR1 (F, *hsd*S20 (r'_{b}, m⁻_{b}), *rec*A⁺, *ara-*14, *pro*A2, *lac* Y1*, gal*K2*, rps*L20 (Sm'), *xyl*-5, *mtl-*1*, sup*E44, λ-), and K802 (*hsdR, gal, met, supE, mcrA, merB*) were used for selection and expression of chimeric genes, respectively.

### Animals

BALB/C (H-2^{d}) female mice were used approximately 7-10 weeks old, weight 20 mg. New Zealand white strain female rabbits were used for obtaining polyclonal antibodies.

### Construction of HBc Derivatives

*Vectors based on plasmids pHBc3 and pHBc16-15.* Vector pHBc3 was constructed by putting the HBc gene under the control of the tandem repeat of *E.coli trp* promoters. Vector pHBc16-15 was constructed by insertion of an oligonucleotide linker carrying *Cla* I/Eco *RV* restriction sites into position 144 of the HBc gene.

*Construction of chimeric HBc derivatives.* The structure of the HBc and HBcΔ derivatives is shown in Table 3. The recombinant genes were constructed by insertion of the appropriate HBV preS1, preS2, and HIV-1 gag fragments into the Cla I site of the pHBc16-15 vector, with or without in-frame junction to the C-terminal part of the HBc gene.

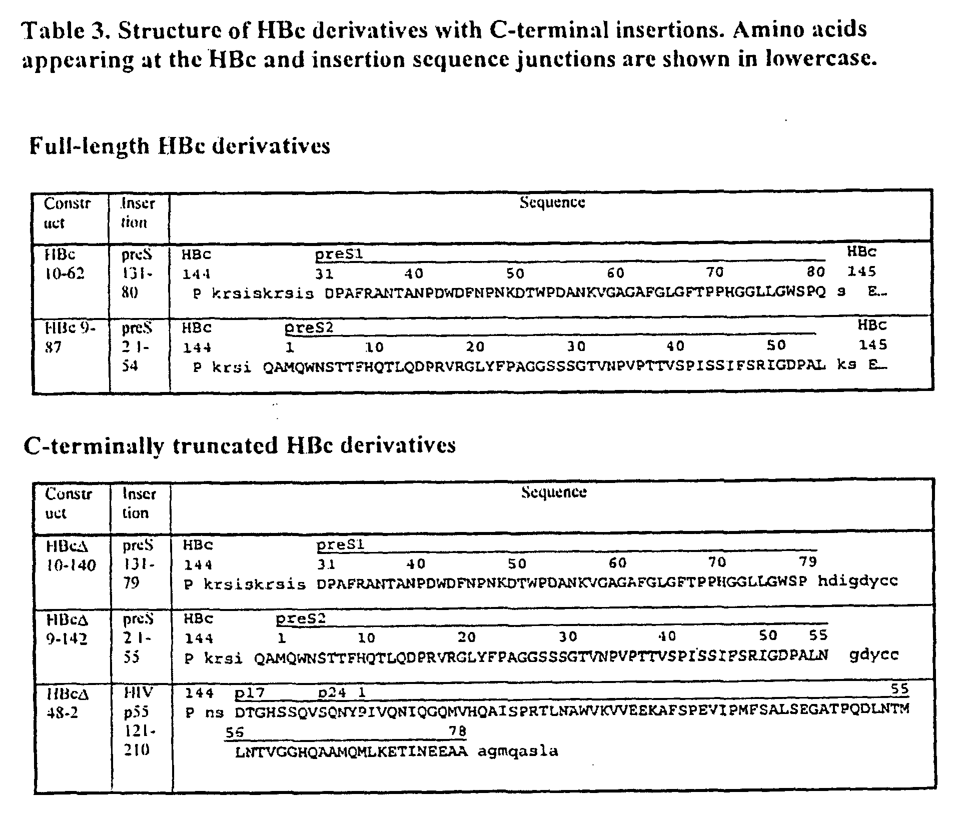

### Purification of Chimeric HBc Derivatives

*E.coli* cells were grown overnight on a rotary shaker at 37°C in 750 ml flasks containing 300 ml of M9 minimal medium supplemented with 1% casamino acids (Difco Laboratories, Sparks, USA) and 0.2% glucose. An optical density OD₅₄₀ of 2-5 was usually reached. Generally, cells were pelleted and lysed by 30 min incubation on ice in lysis buffer containing 50 mM Tris-HCl (pH 8.0), 5 mM EDTA, 50 µg/ml PMSF, 2 mg/ml lysozyme and then ultrasonicated 3 times for 15 s at 22 kHz. Lysates were then adjusted to 10 mM MgCl₂, and 20 µg/ml DNAase. After low speed centrifugation, proteins were precipitated from the supernatant with ammonium sulfate at 33% saturation for 1-2 h at 4°C. Pellets were resuspended in a standard PBS buffer containing 0.1% Triton X-100™, and 5 ml of the solutions were loaded on a Sepharose CL4B™ column (2.5 x 85 cm) and eluted with PBS buffer without Triton X-100. The presence of HBc polypeptides in fractions was tested by PAGE. Positive fractions were pooled and concentrated by ammonium sulfate precipitation at 33% saturation for 20 h at 4°C. Pellets were resuspended in PBS, or in Tris-saline buffer, 10 mM Tris-HCl (pH7.5), 150 mM NaCl, to a final concentration of about 5-20 mg/ml, dialyzed overnight against 2000 volumes of the same buffer and stored at -70°C or at -20°C in 50% glycerol.

### Polyacrylamide Gel Electrophoresis and Western Blotting

For PAGE analysis, bacteria were pelleted, suspended in SDS-gel electrophoresis sample buffer containing 2% SDS and 2% 2-mercaptoethanol and lysed by heating at 100°C for 5 min. The proteins were separated by Laemmli's polyacrylamide gel electrophoresis (PAGE) in a slab gel (150x150x0.75 mm) apparatus with a gradient 12-18% running gel and a 4% stacking gel. Western blotting was performed in general as described by Towbin et al (1979). Nitrocellulose sheets (0.2 µ, Millipore, Bedford, USA) were incubated with anti-HBc antibodies and anti-preS1 antibody in dilutions of 1:100 to 1:1000 overnight and then with anti-mouse IgG peroxidase conjugate (1:1000) for 1-2 h at room temperature. The reaction was developed with 3,3'-diaminobenzidine. In parallel, gels were silver-stained according to Ohsawa and Ebata (1983).

### Immunisations

Mice (five per group) were immunised at day 0 intraperitoneally with 0.02 mg of chimeric particles in complete Freund's adjuvant (CFA, Difco) followed by two booster immunisations in Freund's incomplete adjuvant (IFA, Difco) given at days 10 (0.01 mg intraperitoneally) and 24 (0.01 mg intraperitoneally and 0.01 mg subcutaneously). Sera obtained on day 32 were analysed by ELISA for reactivity with HBc particles.

### ELISA

For the ELISA, recombinant HBc particles were coated onto 96-well microtiter plates by air-drying in a chemical hood overnight. Wells were blocked with 0.5% BSA in PBS for 1 h, incubated with serial dilutions of the various antibodies for 1 h at 37°C and processed with the appropriate second antibodies conjugated to horse radish peroxidase (Sigma) according to the protocols of the manufacturers. Plates were washed 5 times between incubations with 0.05% Tween-20™ in PBS, and 5 times with distilled water to remove Tween-20. Optical absorbances were measured at 492 nm in an automatic Immunoscan MS™ reader. The titres were calculated as the negative logarithms of the EC50 (effective concentration, 50%) serum dilution on the basis of sigmoidal dose-response curves. GraphPad Prism® version 3.02 software was used in the mean titre calculations.

### Results

*Immunogenicity of Recombinant Proteins.* To measure the immunogenicity of HBc carrier and inserted preS1, preS2, and Gag sequences, individual mice sera were repeatedly tested by direct ELISA using recombinant HBcAg and synthetic preS1, preS2, and HIV-1 p24 peptides on solid support. Immunisation with chimeric particles induced high levels of anti-HBc and relatively low levels of anti-insertion antibodies (not shown).

### Induction of Different Immunoglobulin Subclasses by Chimeric HBcΔ-preS1 (20-47) Particles

In order to average obtained immunisation data and to make them more informative for comparative subclass analysis of induced immunoglobulins, we calculated mean titres for each group of immunised animals as the negative logarithms of the EC50 (effective concentration, 50%) serum dilution on the basis of sigmoidal dose-response curves (GraphPad Prism® version 3.02). These data on the anti-HBc response of immunised mice, which allow direct comparison of averaged titres, are given in Fig. 22.

The data presented in Fig. 22 show that the wild type HBcAg induces anti-HBc response with the immunoglobulin subclass distribution IgG2a≥IgG2b>IgG1, whilst the immune response to the C-terminally truncated HBcΔ structure T31 presents the IgG1>IgG2b≥IgG2a subclass distribution pattern. The full-length HBc derivative 10-62, which carries 50 aa long preS1 insertion, shows a subclass distribution analogous to that of the full-length HBc vector. Moreover, replacement of the C-terminus of the HBc molecule by a long foreign insertion (50 aa of the pres 1 sequence) in the HBc derivative 10-140 makes the subclass distribution of the anti-HBc antibodies rather similar to that induced by the full-length HBc structure (Fig. 22). The HBcΔ derivative 48-2 with a 100 aa long insertion of HIV-1 Gag occupies an intermediate position in this sense between wild type HBcAg and C-terminally truncated HBcΔ T31 structures.

### EXAMPLE 6 - Expression and properties of C-terminally truncated HBc derivatives carrying HBV preS1 region 20-47 inserted into or instead of the major immunodominant region (the e1 loop) of the HBc molecule

The experiments described in this Example were carried out in the laboratory of a collaborator of the inventors, Dr Paul Pumpens.

### Summary

A set of C-terminally truncated HBc vectors (HBc-Δ) with deletions of different length within the major immunodominant region (MIR) of the HBc molecule has been constructed. The HBV preS1 region 20-47 was inserted into these vectors, and recombinant HBcΔ-preS1 genes were expressed in *E.coli* cells. The appropriate chimeric HBc-preS1 particles were purified and examined antigenically and immunogenically.

Chimeric HBcΔ-preS1 particles exposed the preS1 region on their outer surface. Immunisation of mice showed high immunogenicity of both the HBc carrier and preS1 epitope parts of the chimeric particles.

Subclass analysis of induced immune responses showed that the immunoglubin subclass distribution of IgGI, IgG2a, IgG2b, IgG3, and IgM antibodies constitutes a ratio IgG1>IgG2a≥IgG2b>IgG3>IgM in the case of the HBc carrier and IgG1≥IgG2a≥IgG2b≥IgG3>IgM in the case of the preS1 epitope. The following arrangement of the anti-HBc response was found for the C-terminally truncated HBcΔ vectors with or without deletions of the MIR sequences, in contrast to the Ig ratio which is typical for the HBc particles with the native C-terminal part: IgG2a>IgG2b>IgG3>IgG1>IgM. Restoration of the native C-terminal part of the HBc molecules within the chimeric HBc-preS1 particles re-established the Ig ratio which is typical for the HBc derivatives with the wild type C-terminus.

### Materials and Methods

### Bacterial Strains

*E.coli* strains RR1 (F, *hsd*S20 (r⁻_{b}, m⁻_{b}), *rec*A⁺, *ara-*14, *pro*A2*, lac*Y1, *gal*K2, *rps*L20 (Sm^{r}), *xyl*-5, *mtl-*1, *sup*E44, λ⁻), and K802 (*hsdR, gal, met, supE, mcrA, mcrB*) were used for selection and expression of chimeric genes, respectively.

### Animals

BALB/C (H-2^{d}) female mice were used for immunisation approximately 7-10 weeks old, weight 20 mg. New Zealand white strain female rabbits were used for obtaining polyclonal antibodies by immunisation in accordance with traditional protocols.

### Peptides

The pres 1 peptide 21-PLGFFPDHQLDPAFRANTANPDWDFNP-47 was used.

### Construction of HBcΔ vectors

*Vectors based on plasmid p2-19.* The construction map of HBc derivatives and their structures are shown in Fig. 23 and Table 4, respectively. The initial p2-19 vector was constructed by introduction of a model preS1 epitope 31-DPAFR-35 surrounded by unique *Eco* 721 and *Eco* 1051 restriction sites between the positions 78 and 79 of the C-terminally truncated HBc gene (Borisova et al., 1999).

For the construction of a set of HBcΔ genes with deletions within the MIR, the p2-19 vector was linearised with *Eco* 721 or *Eco* 1051 restriction nuclease, then shortened by 15-45 nucleotides with *Bal* 31 nuclease (Fermentas, Vilnius, Lithuania), and recircularised with T4 DNA ligase, in the presence of the oligonucleotide encoding the initial preS1 epitope surrounded by the *Eco* 721 and *Eco* 1051 restriction sites, or without it. The in-frame HBc variants were selected by immune screening of bacterial colonies with anti-HBc antibodies. The HBcΔ MIR deletion variant p105-1-21 and HBcΔ vectors pS1-8, pS2-11, and pS2-16 were constructed by this approach (Table 4).

*Vectors p364-15 and 369.* Construction of these vectors is shown schematically in the Fig. 23.

*Construction ofHBcΔ-preS1(20-47) chimeras.* Construction of the HBcΔ-preS1(20-47) variants is shown in Fig. 23, but their structure in Table 4. The synthetic oligonucleotide encoding the preS1 epitope 20-NPLGFFPDHQLDPAFRANTANPDWDFNP-47 surrounded by Eco 72I and Eco 105I restriction sites was inserted into the *Eco* 72I/*Eco* 105I cleaved vectors p2-19 (constructs 343-47, and 343-54), pS1-8 (construct S1-8-3), pS2-11 (construct S2-11-26), and pS2-16 (S2-16-13). The constructs 361-1 and 366, and 341-12 were obtained as shown in Fig. 23.

*Construction of C-terminally complete HBc-preS1 (20-47) chimeras.* The structure of the HBc-preS1 constructs is shown in Table 4. Restoration of native C-terminal part of the HBc derivatives was achieved by addition of the wild type C-terminus to the appropriate HBcΔ-preS1(20-47) chimeras.

### Purification of Chimeric HBc Derivatives

*E.coli* cells were grown overnight on a rotary shaker at 37°C in 750 ml flasks containing 300 ml of M9 minimal medium supplemented with 1% casamino acids (Difco Laboratories, Sparks, USA) and 0.2% glucose. An optical density OD₅₄₀ of 2-5 was usually reached. Generally, cells were pelleted and lysed by 30 min incubation on ice in lysis buffer containing 50 mM Tris-HCl (pH 8.0), 5 mM EDTA, 50 µg/ml PMSF, 2 mg/ml lysozyme and then ultrasonicated 3 times for 15 s at 22 kHz. Lysates were then adjusted to 10 mM MgCl₂ and 20 µg/ml DNAase. After low speed centrifugation, proteins were precipitated from the supernatant with ammonium sulfate at 33% saturation for 1-2 h at 4°C. Pellets were resuspended in a standard PBS buffer containing 0.1% Triton X-100™. 5 ml of the solutions was loaded on a Sepharose CL4B™ column (2.5 x 85 cm) and eluted with PBS buffer without Triton X-100™. The presence of HBc polypeptides in fractions was tested by PAGE. Positive fractions were pooled and concentrated by ammonium sulfate precipitation at 33% saturation for 20 h at 4°C. Pellets were resuspended in PBS, or in Tris-saline buffer, 10 mM Tris-HCl (pH7.5), 150 mM NaCl, to a final concentration of about 5-20 mg/ml, dialyzed overnight against 2000 volumes of the same buffer and stored at -70°C or at -20°C in 50% glycerol.

For purification of S1-8, S2-11, and S2-16 chimeras and their derivatives, lysates were adjusted to 0.1 M urea before low speed centrifugation. For S1-2, the lysate was adjusted to 0.3 M urea before low speed centrifugation. Ammonium sulfate precipitates were washed with PBS buffer, and then dissolved in PBS containing 1.5 M urea, 0.6% Triton X-100™, just before loading onto the Sepharose CL4B™ column. The HBc derivatives were eluted from the column with PBS buffer containing 0.25 M urea and 0.01% Triton X-100™. For purification of the B-20 chimera, lysate was adjusted to 1 M urea and 0.3% Triton X-100™ before low speed centrifugation. The ammonium sulfate precipitate was washed and dissolved as described for the S1-2 chimera. The column buffer contained 0.9 M urea and 0.05% Triton X-100™.

### Polyacrylamide Gel Electrophoresis and Western Blotting

For PAGE analysis, bacteria were pelleted, suspended in SDS-gel electrophoresis sample buffer containing 2% SDS and 2% 2-mercaptoethanol and lysed by heating at 100°C for 5 min. The proteins were separated by Laemmli's polyacrylamide gel electrophoresis (PAGE) in a slab gel (150x150x0.75 mm) apparatus with a gradient 12-18% running gel and a 4% stacking gel.

Western blotting was performed in general as described by Towbin et al. (1979). Nitrocellulose sheets (0.2 µ, Millipore, Bedford, USA) were incubated with anti-HBc antibodies or anti-preS1 antibody in dilutions of 1:100 to 1:1000 overnight and then with anti-mouse IgG peroxidase conjugate (1:1000) for 1-2 h at room temperature. The reaction was developed with 3,3'-diaminobenzidine. In parallel, gels were silver-stained according to Ohsawa and Ebata (1983).

### Immune Electron Microscopy

Colloidal gold immunoelectron microscopic analysis was performed as described elsewhere (Louro and Lesemann, 1984). Goat anti-mouse IgG+IgM and protein A, both labelled with 5-nm colloidal gold particles (TAAB, Berkshire, England), were used for decoration of the HBc derivatives tested with anti-preS1 antibody, and with polyclonal rabbit anti-HBc antibodies, respectively. The grids were examined with a JEM 100C™ electron microscope operated at 80 kV.

### Direct and Competitive ELISA

For the direct ELISA, preS1 peptide 21-47, wild type HBc particles, and chimeric HBc derivatives (20 µg/ml) were coated onto 96-well microtiter plates by air-drying in a chemical hood overnight. Wells were blocked with 0.5% BSA in PBS for 1 h, incubated with serial dilutions of the various antibodies for 1 h at 37°C and processed with the appropriate second antibodies conjugated to horse radish peroxidase (Sigma) according to the protocols of the manufacturers. Plates were washed 5 times between incubations with 0.05% Tween-20™ in PBS, and 5 times with distilled water to remove Tween-20™. Optical absorbances were measured at 492 nm in an automatic Immunoscan MS™ reader. For the competitive ELISA, serial dilutions of antigens were pre-incubated with a standard dilution of the appropriate antibody and the ELISA reaction was continued as described above. The antibody dilution was chosen to yield 50 % of the reaction maximal OD₄₉₂ according to the calibration curve of serial dilutions of the antibody.

### Immunisations

*Standard scheme of immunisation.* Mice (five per group) were immunised at day 0 intraperitoneally with 0.02 mg of chimeric particles in complete Freund's adjuvant (CFA, Difco) followed by two booster immunisations in Freund's incomplete adjuvant (IFA, Difco) given at days 10 (0.01 mg intraperitoneally) and 24 (0.01 mg intraperitoneally and 0.01 mg subcutaneously). Sera obtained on day 32 were analysed by ELISA for reactivity with HBcAg particles and with preS1 peptide 21-47.

*Long scheme of immunisation.* Mice (five per group) were immunised at day 0 intraperitoneally with 0.1 mg of chimeric particles in CFA, followed by three booster immunisations of 0.05 mg of particles in IFA intraperitoneally at days 42, 56, and 63. Sera were analysed by ELISA on day 66.

*Short scheme of immunisation.* Mice (five per group) were immunised at days 0 and 8 intraperitoneally with 0.2 mg of chimeric particles in CFA, followed by three booster immunisations in IFA given at days 12 (0.1 mg intravenously and 0.1 mg subcutaneously), 13 (0.2 mg intraperitoneally), and 14 (0.1 mg intraperitoneally and 0.1 mg intravenously). Sera obtained on day 15 were analysed by ELISA.
*Calculation of titres.* The titres were calculated as the negative logarithms of the EC50 (effective concentration, 50%) serum dilution on the basis of sigmoidal dose-response curves. GraphPad Prism® version 3.02 software was used in the mean titre calculations.

### Results

### Antigenicity of Chimeric HBcΔ-preS1(20-47) Particles

HBcΔ-preS1(20-47) derivatives behaved as preS1-presenting in immunogold electron microscopy (not shown). The actual analysis of internal or superficial location of foreign sequences was performed however by competitive ELISA, where the chimeric particles and antibody targeted to the inserted preS1 epitope were allowed to interact in solution prior to exposure to the appropriate preS1(21-47) peptide bound on solid phase. Competitive ELISA showed that a minimal difference in the competitive capacity of different HBcΔ-preS1 (20-47) derivatives in comparison with the free preS1 (20-47) peptide and established therefore the full superficial accessibility of the inserted preS1 region.

### Immunogenicity of Chimeric HBcΔ-preS1 (20-47) Particles

To measure the immunogenicity of HBc carrier and inserted preS1 sequence, individual mice sera were repeatedly tested by direct ELISA technique using recombinant HBcAg and synthetic preS1(21-47) peptide on solid support, respectively.

Immunisation with chimeric particles induced significant levels of anti-HBc as well as anti-preS1 antibodies in mice. Table 5 shows individual anti-HBc and anti-preS1 responses of mice, where the appropriate titres are calculated in accordance with a traditional approach. Namely, the antibody titers are defined here as decimal logarithms of reciprocal serum dilutions corresponding to three of the mean of the background (sera of non-immunised mice) absorbance values. The insertion of preS1(20-47) into the MIR of the HBc protein provides an extremely high antibody response to the preS1 sequence, which does not differ from the original anti-HBc responses. No significant differences in the anti-preS1 induction capacities were found for chimeric HBcΔ derivatives carrying different deletions within the MIR of the HBc protein. The long immunisation scheme led to higher anti-preS1 titres in comparison to short and traditional schemes of immunisation (Table 5).

### Induction of Different Immunoglobulin Subclasses by Chimeric HBcΔ-preS1 (20-47) Particles

In order to average the immunisation data obtained and to make them more informative to comparative subclass analysis of induced immunoglobulins, we calculated mean titres for each group of immunised animals as the negative logarithms of the EC50 (effective concentration, 50%) serum dilution on the basis of sigmoidal dose-response curves (GraphPad Prism® version 3.02). These data on anti-HBc and anti-preS1 response of immunised mice, which allow direct comparison of averaged titres, are given in Figs 24A and 24B respectively.

The data presented in the Fig. 24A show that the HBcΔ-preS1(20-47) constructs follow in general the immunoglobulin subtype distribution of IgG1>IgG2a≥IgG2b>IgG3>IgM, which is typical for the C-terminally truncated HBcΔ vectors. The clear exception is presented by the 364-15 vector structure, which remains rather similar to the full-length HBc structure (Fig. 24A). However, the HBcΔ-preS1(20-47) construct 366, created from 364-15, shows the subclass pattern which is typical of the HBcΔ derivatives.

The anti-preS1 response to C-terminally truncated HBcΔ-preS1 (20-47) constructs follows the immunoglobulin subtype distribution of IgG1≥IgG2a≥IgG2b≥IgG3>IgM, with the exception of the HBcΔ-preS1(20-47) construct 366, which shows clearly an IgG1 prevalent response (Fig. 24B).

### Restoration of the Subclass Pattern by Addition of the C-terminus to the HBcΔ Constructs

The subclass ratio IgG2a>IgG2b>IgG3>IgG1>IgM, which is typical for HBc particles harbouring HBc monomers with the native C-terminal part, was restored by addition of the natural C-terminus to the HBcΔ-preS1(20-47) constructs (see the results for constructs 391, 392, 393 and 394 in Fig. 24). This suggests that the C-terminus of HBc, and in particular the C-terminal cysteine, is important for Th1 induction.

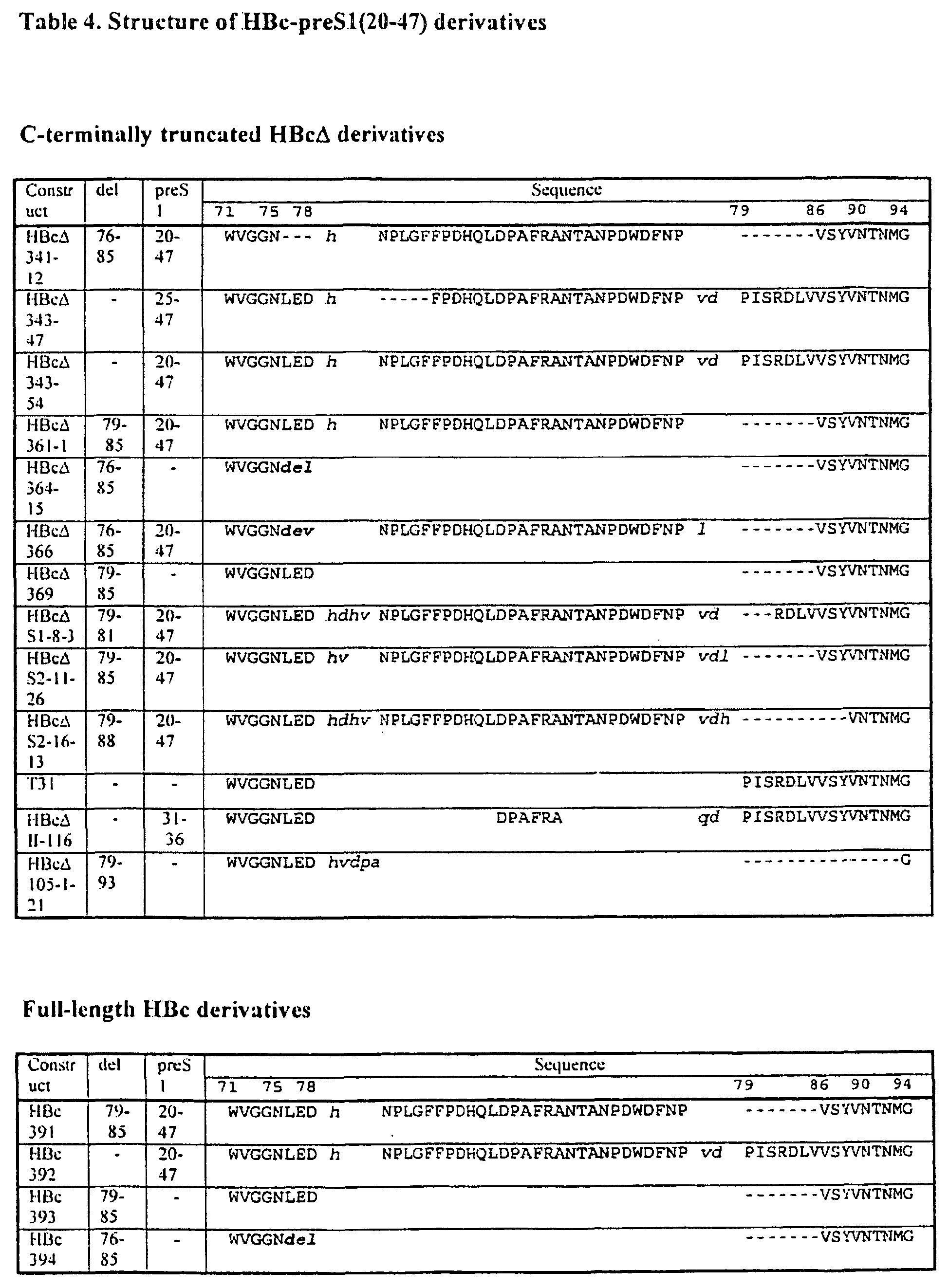

Table 5. Individual anti-HBc and anti-preS1 responses of immunised mice. The anti-HBc and anti-preS1 titers are calculated as logarithms of reciprocal serum dilutions corresponding to the mean (n=3) of the background (sera of non-immunised mice) absorbance values.

| HBc | | | | | | |
|---|---|---|---|---|---|---|
| | Total | IgG1 | IgG2a | IgG2b | IgG3 | IgM |
| 341-12-1 | 4,53 | 4,32 | 3,81 | 3,85 | 0,00 | 0,00 |
| 341-12-2 | 4,72 | 4,73 | 3,40 | 3,81 | 2,89 | 0,00 |
| 341-12-3 | 4,32 | 5,01 | 4,29 | 3,73 | 2,48 | 2,48 |
| 341-12-4 | 4,59 | 5,31 | 3,81 | 3,37 | 3,32 | 2,41 |
| 361-1-1 | 5,30 | 5,34 | 4,73 | 4,83 | 3,37 | 2,38 |
| 361-1-2 | 5,16 | 5,24 | 4,59 | 3,99 | 0,00 | 2,30 |
| 361-1-3 | 4,34 | 4,36 | 3,81 | 3,99 | 2,62 | 2,68 |
| 343-54-1 | 3,85 | 3,85 | 3,69 | 3,30 | 0,00 | 0,00 |
| 343-54-2 | 4,15 | 4,73 | 4,21 | 4,39 | 3,58 | 3,37 |
| 343-54-3 | 4,76 | 4,86 | 3,85 | 3,40 | 3,51 | 2,82 |
| 343-54-4 | 4,34 | 4,73 | 4,86 | 4,64 | 3,32 | 0,00 |
| 343-47-1 | 4,86 | 5,12 | 4,25 | 3,77 | 2,89 | 2,82 |
| 343-47-2 | 4,76 | 5,34 | 4,32 | 4,21 | 3,85 | 3,26 |
| 343-47-3 | 4,38 | 4,32 | 3,37 | 3,77 | 3,40 | 2,34 |
| 343-47-4 | 4,76 | 4,64 | 3,91 | 3,29 | 3,64 | 3,43 |
| 366short-1 | 4,32 | 5,28 | 3,69 | 4,11 | 2,95 | 3,33 |
| 366short-2 | 4,63 | 4,46 | 3,88 | 3,37 | 3,26 | 2,30 |
| 366long-1 | 4,86 | 6,38 | 4,69 | 4,73 | 4,11 | 3,30 |
| 366-long-2 | 4,53 | 5,31 | 4,73 | 4,73 | 3,88 | 3,03 |
| 105 long | 5,79 | 5,82 | 5,72 | 5,72 | 5,16 | 3,91 |
| 105 short | 4,20 | 4,39 | 3,40 | 3,91 | 3,03 | 3,81 |
| HBcAg-1 | 5,20 | 4,73 | 5,82 | 5,31 | 5,07 | 0,00 |
| HBcAg-2 | 5,30 | 3,64 | 5,72 | 4,29 | 4,32 | 3,21 |
| HBcAg-3 | 5,20 | 4,53 | 5,28 | 4,73 | 4,16 | 3,26 |
| HBcAg-4 | 5,48 | 4,69 | 5,82 | 5,28 | 4,94 | 3,85 |
| HBcAg-5 | 5,11 | 4,36 | 5,34 | 4,83 | 4,73 | 3,58 |
| HBcAg-3 D | 5,82 | 5,01 | 4,64 | 5,16 | 4,83 | 3,88 |
| HBcAg-4 D | 5,22 | 4,77 | 4,36 | 5,12 | 4,80 | 3,85 |
| HBcAg-5 D | 5,31 | 4,69 | 4,73 | 4,98 | 4,69 | 3,77 |
| pT31-1 | 5,16 | 4,69 | 3,81 | 4,16 | 4,29 | 3,85 |
| pT31-2 | 5,00 | 4,77 | 3,40 | 3,91 | 3,88 | 4,11 |
| pT31-3 | 5,62 | 5,07 | 4,21 | 4,32 | 4,59 | 4,16 |
| pT31-4 | 5,07 | 4,80 | 4,29 | 4,25 | 4,39 | 4,11 |
| pT31-5 | 5,22 | 4,94 | 3,88 | 4,16 | 4,39 | 4,11 |
| 369-1-1 | 4,69 | 4,25 | 3,91 | 3,99 | 3,37 | 2,73 |
| 369-1-2 | 4,53 | 4,39 | 3,40 | 4,29 | 2,68 | 2,38 |
| 369-1-3 | 4,39 | 4,77 | 3,26 | 3,40 | 2,97 | 3,51 |
| 369-1-4 | 4,83 | 4,94 | 4,39 | 4,29 | 3,10 | 2,78 |
| 369-1-5 | 4,64 | 4,36 | 4,05 | 4,59 | 3,81 | 2,92 |
| 364-15-1 | 4.83 | 4,16 | 4,29 | 4,39 | 3,58 | 0,00 |
| 364-15-2 | 4,62 | 4,32 | 3,81 | 3,91 | 3.40 | 2,89 |
| 364-15-3 | 4,36 | 3,91 | 3,77 | 3,77 | 3,64 | 2,73 |
| 364-15-4 | 4,25 | 3,85 | 3,40 | 3,40 | 3,03 | 2,73 |

| preS1 | | | | | | |
|---|---|---|---|---|---|---|
| | Total | IgG1 | IgG2a | IgG2b | IgG3 | Igm |
| 341-12-1 | 4,63 | 4,69 | 4,05 | 4,21 | 3,58 | 0,00 |
| 341-12-2 | 4,68 | 4,83 | 4,36 | 3,77 | 3,51 | 0,00 |
| 341-12-3 | 4,72 | 4,36 | 4,25 | 3,69 | 2,38 | 2,71 |
| 341-12-4 | 3,91 | 4,31 | 3,30 | 2,82 | 0,00 | 2,56 |
| 361-1-1 | 5,59 | 5,16 | 4,73 | 4,64 | 4,21 | 0,00 |
| 361-1-2 | 5,01 | 4,86 | 4,46 | 4,21 | 2,89 | 2,34 |
| 361-1-3 | 5,11 | 4,80 | 4,69 | 4,83 | 4,21 | 3,03 |
| 343-54-1 | 4,59 | 4,31 | 3,81 | 3.47 | 2,62 | 2,89 |
| 343-54-2 | 4,76 | 4,64 | 4,32 | 4,36 | 4,80 | 2.45 |
| 343-54-3 | 4,80 | 4,73 | 4,32 | 3,30 | 4,77 | 2,62 |
| 343-54-4 | 5,31 | 4,94 | 4,83 | 4,39 | 4,86 | 2,92 |
| 343-47-1 | 4,86 | 4,94 | 4.46 | 4,39 | 3,81 | 0,00 |
| 343-47-2 | 5,04 | 5,07 | 4.83 | 4,73 | 4,25 | 2,86 |
| 343-47-3 | 5,23 | 4,64 | 4.64 | 4,83 | 4,83 | 3,30 |
| 343-47-4 | 5,11 | 4,80 | 4,59 | 4,83 | 4,64 | 2,00 |
| 366short-1 | 4,23 | 4,36 | 3.43 | 3,40 | 3,58 | 2,95 |
| 366short-2 | 4,28 | 5,60 | 4,53 | 3,30 | 4,11 | 3,26 |
| 366long-1 | 5,53 | 5,82 | 4,83 | 4,53 | 4,69 | 2,48 |
| 366-long-2 | 5,32 | 5,82 | 4,86 | 4,59 | 5,07 | 2,76 |

**Table 6. Summary of results from the Examples**

| | Forms particles? | Contains DNA? | Endotoxin content (EU/ml) | Contains C-terminal Cys? | Th dominance of response against the e1 loop or inserted epitope | Th dominance of response against the non-e1 loop regions |
|---|---|---|---|---|---|---|
| Core-S1 | Yes | Yes | 808 | Yes | Th1 | Th2 |
| Core-S1₁₅₄ | Mixed | No | 1182 | No | Th2 | Th2 |
| Core | Yes | Yes | ND | Yes | Th1 | Th1 |
| Core₁₄₆ | Mixed | No | ND | No | Th2 | Th2 |
| Core-S1₁₄₆(a) | Yes | ND | ND | No | Th2 | ND |
| Hepacore PS2 (b) | ND | ND | ND | Yes | Th1 | Th1 |
| HBc 10-62 (c) | ND | ND | ND | Yes | Th1 | ND |
| HBc 10-140 (d) | ND | ND | ND | Yes | Th1 | ND |
| HBcΔ-preS1 products of Ex 6 | ND | ND | ND | No | Th2 | Th2 |
| HBcΔ-preS1 products of Ex 6 with C-terminus restored | ND | ND | ND | Yes | Th1 | Th1 |

| | | | | | | |
|---|---|---|---|---|---|---|
| ND = not determined | | | | | | |
| (a) = provided by a collaborator, Dr Paul Pumpens | | | | | | |
| (b) = full length HBc with a pre-S2 insert in the e1 loop | | | | | | |
| (c) = full length HBc with a 50aa pre-S1 insert in the C-terminus | | | | | | |
| (d) = HBc with a deletion in the C-terminus and replacement of the deleted sequence with 50 aa of pre-S1 sequence | | | | | | |

### REFERENCES

Birnbaum, F & Nassal, M (1990). Hepatitis B virus nucleocapsid assembly: primary structure requirements in the core protein. *J. Virology* **64**, 3319-3330.

Borisova, G., O. Borschukova, D. Skrastina, A. Dislers, V. Ose, P. Pumpens and E. Grens. 1999. Behavior of a short preS1 epitope on the surface of hepatitis B core particles. Biol. Chem. *380*:315-24.

Davis, HL, Weeratna R, Waldschmidt, TJ, Tygrett, L, Schorr J & Krieg, AM. (1998). CpG DNA is a potent enhancer of specific immunity in mice immunized with recombinant hepatitis B surface antigen. *J Immunology* **160**, 870-876.

Lipford, GB, Bauer, M, Blank C, Reiter, R, Wagner, H & Heeg, K. (1997). CpG-containing synthetic oligonucleotides promote B and cytotoxic T cell responses to protein antigen: a new class of vaccine adjuvants. *European J. Immunology* **27**, 2340-2344.

Ohsawa, K., and Ebata, N. (1983). Silver stain for detecting 10-fentogram quantities of protein after polyacrylamide gel electrophoresis. Anal. Biochem. *135*, 409-415.

Sambrook, J, Fritsch, EF & Maniatis, T. (1989). Molecular cloning - A laboratory manual (second edition). Published by Cold Spring Harbor Laboratory Press.

Sato, Y, Roman, M, Tighe, H, Lee, D, Corr, M, Nguyen, M-D, Silverman G.J., Lotz, M, Carson, DA & Raz, E. (1996). Immunostimulatory DNA sequences necessary for effective intradermal gene immunization. Science **273**, 352-354.

Sonehara, K, Saito H, Kuramoto, E, Yamamoto, S, Yamamoto, T & Tokunaga, T. (1996). Hexamer palindromic oligonucleotides with 5'-CG-3' motif(s) induce production of interferon. *J Interferon Cytokine Res.* **16,** 799-803.

Towbin, H., Staehelin, T., and Gordon, J. (1979). Electrophoretic transfer of proteins from polyacrylamide gels to nitrocellulose sheets: procedure and some applications. Proc. Natl. Acad. Sci. USA *76*, 4350-4354.

## Claims

1. Use of insertion of a heterologous epitope into a carrier protein and determination of whether the carrier protein induces a Th1 or a Th2 response in the design of an immunogen, wherein the carrier protein is hepatitis B virus core antigen (HBcAg) and the insertion is in the e1 loop of HBcAg.

2. A use according to claim 1 wherein the insertion of the heterologous epitope switches the response against the carrier protein from a Th1 response to a Th2 response.

3. A use according to claim 1 or 2 wherein the heterologous epitope is from a pathogenic organism, from a tumour antigen or from an allergen.

4. A use according to claim 3 wherein the epitope is from a virus.

5. A use according to claim 4 wherein the epitope is from hepatitis B virus (HBV) or hepatitis C virus (HCV).

6. A use according to claim 5 wherein the epitope is from the pre-S1 or pre-S2 sequence of HBV.

7. A use according to any one of the preceding claims wherein the carrier protein with the insertion of the heterologous epitope is effective in immunotherapy of a disease.

8. A use according to claim 7 wherein the carrier protein with the insertion of the heterologous epitope is effective in the treatment of chronic viral hepatitis.

9. A use according to claim 8 wherein the carrier protein with the insertion of the heterologous epitope is effective in the treatment of chronic HBV infection.

## Patentansprüche

1. Anwenden einer Insertion eines heterologen Epitops in ein Trägerprotein und Bestimmen, ob das Trägerprotein eine Th1- oder eine Th2-Reaktion im Design eines Immunogens herbeiführt, wobei das Trägerprotein Hepatitis-B-Virus-Core-Antigen (HbcAg) ist und die Insertion in der E1-Schleife von HbcAg ist.

2. Anwendung nach Anspruch 1, wobei die Insertion des heterologen Epitops die Reaktion gegen das Trägerprotein von einer Th1-Reaktionstage zu einer Th2-Reaktion verlagert.

3. Anwendung nach Anspruch 1 oder 2, wobei das heterologe Epitop von einem pathogenen Organismus, von einem Tumorantigen oder von einem Allergen stammt.

4. Anwendung nach Anspruch 3, wobei das Epitop von einem Virus stammt.

5. Anwendung nach Anspruch 4, wobei das Epitop von Hepatitis-B-Virus (HBV) oder Hepatitis-C-Virus (HCV) stammt.

6. Anwendung nach Anspruch 5, wobei das Epitop von der prä-S1- oder prä-S2-Sequenz von HBV stammt.

7. Anwendung nach einem der vorangehenden Ansprüche, wobei das Trägerprotein mit der Insertion des heterologen Epitops in der Immuntherapie einer Erkrankung wirksam ist.

8. Anwendung nach Anspruch 7, wobei das Trägerprotein mit der Insertion des heterologen Epitops in der Behandlung einer chronischen viralen Hepatitis wirksam ist.

9. Anwendung nach Anspruch 8, wobei das Trägerprotein mit der Insertion des heterologen Epitops in der Behandlung einer chronischen HBV-Infektion wirksam ist.

## Revendications

1. Utilisation d'une insertion d'un épitope hétérologue dans une protéine porteuse et détermination du fait que la protéine porteuse induit une réponse Th1 ou Th2 dans la conception d'un immunogène, dans laquelle la protéine porteuse est un antigène de nucléocapside du virus de l'hépatite B (HBcAg, antigène HBc) et l'insertion est dans la boucle e1 de HBcAg.

2. Utilisation selon la revendication 1 dans laquelle l'insertion de l'épitope hétérologue change là réponse contre la protéine porteuse d'une réponse Th1 en une réponse Th2.

3. Utilisation selon la revendication 1 ou 2 dans laquelle l'épitope hétérologue provient d'un organisme pathogène, d'un antigène de tumeur ou d'un allergène.

4. Utilisation selon la revendication 3 dans laquelle l'épitope provient d'un virus.

5. Utilisation selon la revendication 4 dans laquelle l'épitope provient du virus de l'hépatite B (HBV) ou du virus de l'hépatite C (HCV).

6. Utilisation selon la revendication 5 dans laquelle l'épitope provient de la séquence pré-S1 ou pré-S2 de HBV.

7. Utilisation selon l'une quelconque des revendications précédentes dans laquelle la protéine porteuse avec l'insertion de l'épitope hétérologue est efficace dans l'immunothérapie d'une maladie.

8. Utilisation selon la revendication 7 dans laquelle la protéine porteuse avec l'insertion de l'épitope hétérologue est efficace dans le traitement de l'hépatite virale chronique.

9. Utilisation selon la revendication 8 dans laquelle la protéine porteuse avec l'insertion de l'épitope hétérologue est efficace dans le traitement d'une infection chronique par HBV.
